# EUROPÄISCHE PATENTSCHRIFT

(11) **EP 0 857 211 B1**
(45) Veröffentlichungstag und Bekanntmachung des Hinweises auf die Patenterteilung: **28.02.2007**
(21) Anmeldenummer: 96937224.2
(22) Anmeldetag: 24.10.1996
(51) Int. Cl.: C12N 15/12, C12N 15/85, C07K 14/47, C12Q 1/68

(54) **STARKER HOMOLOGER PROMOTOR AUS HAMSTER**
INTENSIVE HOMOLOGOUS PROMOTER OBTAINED FROM HAMSTERS
PROMOTEUR HOMOLOGUE PUISSANT OBTENU A PARTIR DE HAMSTERS

(30) Priorität: 24.10.1995 DE 19539493
(43) Veröffentlichungstag der Anmeldung: 12.08.1998
(60) Teilanmeldung: 06126019.6
(73) Patentinhaber: Boehringer Ingelheim Pharma GmbH & Co.KG, 55216 Ingelheim am Rhein (DE)
(72) Erfinder: ENENKEL, Barbara, D-88447 Warthausen (DE); GANNON, Frank, D-69115 Heidelberg (DE); BERGEMANN, Klaus, D-88400 Biberach (DE); NOE, Wolfgang, D-88400 Biberach (DE)
(74) Vertreter: Kläs, Heinz-Gerd
(86) Internationale Anmeldenummer: PCT/EP1996/004631
(87) Internationale Veröffentlichungsnummer: WO 1997/015664

(56) Entgegenhaltungen:
- BIOCHIMICA BIOPHYSICA ACTA, Bd. 1204, 1994, ELSEVIER SCIENCE, AMSTERDAM, NL, Seiten 271-278, XP000644352 M. NENOI ET AL.: "Novel structure of a Chinese hamster polyubiquitin gene "
- NATURE, Bd. 328, 1987, MACMILLAN JOURNALS LTD., LONDON,UK, Seiten 823-827, XP002024857 A. ARTISHEVSKY ET AL.: "Cell-cycle regulatory sequences in a hamster histone promoter and their interactions with cellular factors"
- EUR. J. BIOCHEM., Bd. 220, Nr. 2, März 1994, SPRINGER, BERLIN, D, Seiten 395-402, XP000664346 H.A.R. BLUYSSEN ET AL.: "The interferon-stimulated gene 54K promoter contains two adjacent functional interferon-stimulated response elements of different strength, which act synergistically for maximal interferon-alpha inducibility"
- J. BIOL. CHEM., Bd. 260, Nr. 12, 25.Juni 1985, AM. SOC. BIOCHEM. MOL.BIOL.,INC.,BALTIMORE,US, Seiten 7609-7613, XP002024621 P.K. LUND ET AL.: "Nucleotide sequence analysis of a cDNA encoding human ubiquitin reveals that ubiquitin is synthesized as a precursor"
- BIOCHEMICAL AND BIOPHYSICAL RESEARCH COMMUNICATIONS 215 (2). 1995. 682-690. ISSN: 0006-291X, 13.Oktober 1995, XP002024622 CHAN Y-L ET AL: "The carboxyl extensions of two rat ubiquitin fusion proteins are ribosomal proteins S27a and L40."
- GENE, Bd. 107, 1991, ELSEVIER SCIENCE PUBLISHERS,B.V.,AMSTERDAM,NL;, Seiten 205-212, XP002024623 K. CHEN AND I. RUBENSTEIN: "Characterization of the structure and transcription of an ubiquitin fusion gene from maize"
- EMBO J., Bd. 6, Nr. 5, Mai 1987, OXFORD UNIVERSITY PRESS,GB;, Seiten 1429-1439, XP002024624 E. ÖZKAYNAK ET AL.: "The yeast ubiquitin genes: a family of natural gene fusions"
- J. BIOL. CHEM., Bd. 268, Nr. 26, 15.September 1993, AM. SOC. BIOCHEM. MOL.BIOL.,INC.,BALTIMORE,US, Seiten 19545-19551, XP002024625 D. JONES AND E.P. CANDIDO: "Novel ubiquitin-like ribosomal protein fusion genes from the nematodes Caenorhabditis elegans and Caenorhabditis briggsae"
- NATURE, Bd. 338, 30.März 1989, MACMILLAN JOURNALS LTD., LONDON,UK, Seiten 438-440, XP002024626 K.L. REDMAN AND M. RECHSTEINER: "Identification of the long ubiquitin extension as ribosomal protein S27a" in der Anmeldung erwähnt
- BRITISH J. CANCER, Bd. 65, 1992, MACMILLIAN PRESS, LTD., LONDON UK, Seiten 65-71, XP000615669 S.M. ADAMS ET AL.: "Differential expression of translation-association genes in benign and malignant tumours" in der Anmeldung erwähnt
- CANCER RES 53 (8). 1993. 1916-1920. CODEN: CNREA8 ISSN: 0008-5472, 15.April 1993, XP002024627 WONG J M ET AL: "UBIQUITIN RIBOSOMAL PROTEIN S27A GENE OVEREXPRESSED IN HUMAN COLORECTAL CARCINOMA IS AN EARLY GROWTH RESPONSE GENE." in der Anmeldung erwähnt
- FEBS LETTERS, Bd. 322, Nr. 3, Mai 1993, ELSEVIER, AMSTERDAM, NL, Seiten 235-239, XP002024628 N. SHIMBARA ET AL.: "Down-regulation of ubiquitin gene expression during differentiation of human leukemia cells" in der Anmeldung erwähnt
- GENE, Bd. 110, 1992, ELSEVIER SCIENCE PUBLISHERS,B.V.,AMSTERDAM,NL;, Seiten 129-130, XP002024629 M. BOSHART ET AL.: "Reporter constructs with low background activity utilizing the cat gene" in der Anmeldung erwähnt

## Beschreibung

Die Erfindung betrifft einen starken homologen Promotor aus Hamster, Nukleinsäuren, die Promotor- und/oder regulatorische Sequenzen des Ubiquitin-S27a-Gens enthalten, sowie Verfahren zur Herstellung heterologer Genprodukte unter Verwendung solcher Nukleinsäuren.

Bei der wirtschaftlich bedeutenden Herstellung von Proteinen durch Expression rekombinanter Gene in eukaryontischen Wirtszellen, beispielsweise CHO-Zellen, werden bisher heterologe Expressionssysteme verwendet, d.h. daß zum Beispiel die Promotor/Enhancer- sowie Terminationselemente viralen Ursprungs sind. Die Verwendung von nichtviralen Promotoren an Stelle von viralen Sequenzen in Expressionssystemen wäre im Hinblick auf die Einstellung der Öffentlichkeit bezüglich Gen- und Biotechnologie von Vorteil und würde außerdem zur Biosicherheit der Vektorsysteme, die fur die Expression von Genen in tierischen Zellkulturen verwendet werden, beitragen.

Beim Ubiquitin handelt es sich um ein hoch konserviertes Polypeptid von 76 Aminosäuren, das in allen eukaryontischen Zellen in großer Zahl zu finden ist und durch eine diverse Genfamilie kodiert wird (Reviews: Jentsch et al., 1991; Schlesinger & Bond, 1987). Ubiquitin spielt durch die Modifizierung von Targetproteinen eine bedeutende Rolle in einer Vielzahl von biologischen Prozessen, wie z.B. der ATP-abhängigen Proteindegradation über den Ubiquitin-Proteosomen-Weg (Ciechanover, 1994). Auf Grund ihrer Struktur kann man die Ubiquitingene in zwei Gruppen einteilen. In der ersten Gruppe sind die Polyubiquitingene zusammengefaßt, in denen 228 bp große Ubiquitin-Kodiereinheiten (= 76 Aminosäuren) in Kopf-zu-Schwanzanordnung aneinandergereiht sind (Jentsch et al., 1991; Schlesinger & Bond, 1987). Die Anzahl der Einheiten variiert von Spezies zu Spezies, wobei man in den meisten Organismen zwei Polyubiquitingene unterschiedlicher Länge findet (Fornace et al., 1989; Wiborg et al., 1985). Die Promotorregionen dieser Gene enthalten eine TATA-Box und Promotor/Enhancer-Elemente für einen Hitzeschockinduktor (Schlesinger & Bond, 1987).

Die Ubiquitinfusionsgene werden der zweiten Gruppe zugeordnet. Es handelt sich dabei um Fusionen zwischen einer Ubiquitineinheit und einem ribosomalen Protein (Jentsch et al., 1991; Schlesinger & Bond, 1987). Die beiden bekannten Ubiquitinfusionsgene können auf Grund der Unterschiede in der Länge und Sequenz des ribosomalen Proteins identifiziert werden. In dem einen Fall handelt es sich um ein ribosomales Protein der großen Ribosomenuntereinheit mit einer Länge von 52 Aminosäuren (Baker et al., 1991) und in dem anderen Fall um ein ribosomales Protein der kleinen Ribosomenuntereinheit (in Säugern als Protein S27a bezeichnet) mit einer speziesabhängigen Länge von 76 bis 81 Aminosäuren (Redman & Rechsteiner, 1989). Der Ubiquitinanteil in diesen Fusionsproteinen unterstützt dabei anscheinend die effiziente Integration der ribosomalen Proteine in das Ribosom (Finley et al., 1989).

Die individuellen Ubiquitingene werden in den verschiedensten Geweben eines Organismus und in verschiedenen Entwicklungsstadien in unterschiedlichem Ausmaß exprimiert. So werden die Polyubiquitingene konstitutiv in geringem Level, der lediglich unter Streßeinwirkung stark erhöht wird, exprimiert (Fornace et al., 1989; Schlesinger & Bond, 1987). Die Ubiquitinfusionsgene werden vor allem verstärkt in der exponentiellen Wachstumsphase exprimiert. In terminal differenzierten und wachstumsarretierten Zellen ist die Expression hingegen herunterreguliert (Schlesinger & Bond, 1987; Shimbara et al., 1993; Wong et al., I 993).

Die Aufgabe, deren Lösung der Gegenstand der vorliegenden Erfindung ist, war die Bereitstellung starker nichtviraler Promotoren für Verfahren zur Herstellung heterologer Genprodukte in Kulturzellen, insbesondere Hamsterzellen.

Überraschend wurde ein Promotor eines Gens aufgefunden, der insbesondere in CHO-Zellen eine dem viralen SV40-Promotor gleichwertige Aktivität hat. Dieses Gen kodiert für das Ubiquitinfusionsprotein Ub/S27a. Der Ub/S27a-Promotor aus Hamster ist Gegenstand der Erfindung.

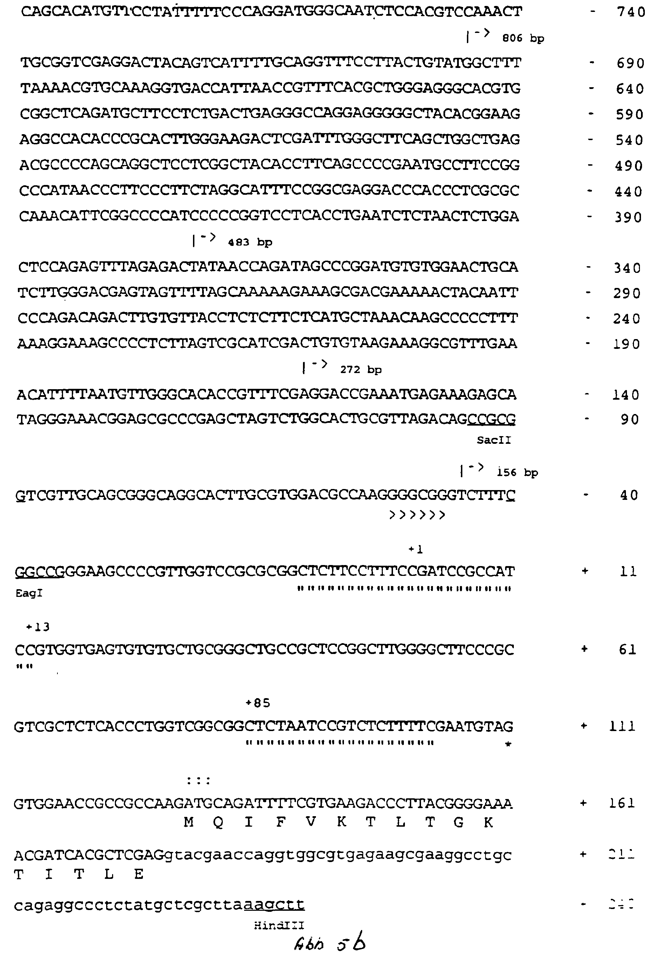

Ein weiterer Gegenstand der vorliegenden Erfindung ist ein Nukleinsäuremolekül, das Promotorsequenzen und/oder andere regulatorische Sequenzen des Ub/S27a-Gens enthält. Dabei sind die Promotorsequenzen und/oder anderen regulatoriscfien Sequenzen aus dem Ub/27a-Gen des Hamsters abgeleitet. Insbesondere ist ein rekombinantes Nukleinsäuremolekül Gegenstand der Erfindung, das Promotorsequenzen und/oder andere regulatorische Sequenzen enthält, die in der Sequenz gemäß Abb. 5 enthalten sind.

Ein bevorzugter Gegenstand der Erfindung sind Nukleinsäuremoleküle, die Sequenzen aus dem Bereich enthalten, der den Positionen -161 bis -45 gemäß Abb. 5 entspricht. Es liegt im Können des Fachmanns, mit den Methoden, die in Beispiel 4 beschrieben werden, Nukleinsäuremoleküle herzustellen, die Teilsequenzen der erfindungsgemäßen Sequenzen enthalten, insbesondere eine Teilsequenz des Bereichs von -161 bis -45, die ebenfalls starke Promotoraktivität vermitteln können. Solche Teilsequenzen sind daher ebenfalls Gegenstand der Erfindung.

Es liegt auch im Können des Fachmanns die Promotorsequenz durch Substitution, Insertion, Deletion oder Addition von einer, zwei oder drei Basen gegenüber der Sequenz gemäß Abb. 5 zu verändern, ohne dadurch die Promotoraktivität, die mit der in Beispiel 4 beschriebenen Methode gemessen werden kann, deutlich zu erniedrigen. Unter einer deutlichen Erniedrigung der Promotoraktivität soll eine Erniedrigung um mehr als 50% des Wertes, der für das 272 bp-Deletionsfragment aus Tabelle 1 im CAT-Assay gemäß Beispiel 4 unter vergleichbaren Bedingungen erhalten wird, verstanden werden. Solche Varianten der Promotorsequenz sind daher im Umfang der Patentansprüche in die Erfindung eingeschlossen.

In den erfindungsgemäßen Nukleinsäuremolekülen sind die Promotor- und/oder regulatorischen Sequenzen vorteilhaft mit einem Gen funktionell verknüpft, sodaß dieses Gen unter der Kontrolle dieser Sequenzen exprimiert werden kann. Ein solches Gen kann beispielsweise für Gewebeplasminogenaktivator (EP 0093619), ein *second-generation-*Plasminogenaktivator, z.B. tnk-t-PA (WO 93/24635), Interferon, z.B. Interferon-α (EP 0595241), Interferon-β (EP 0041313, EP 0287075), Interferon-γ (EP 0146354) oder Interferon-ω (EP 0170204), Tumomekrosefaktor (EP 0168214), Erythropoietin (WO 86/03520), Granulozyten-Kolonie-stimulierenden Faktor (EP 0220520, EP 0237545) oder Mangan-Superoxiddismutase (EP 0282899) kodieren, die aus dem Stand der Technik bekannt sind. Es kann sich auch um ein Gen handeln, das für eine Immunglobulinkette, die variable Domäne einer Immunglobulinkette, einen humanisierten Antikörper (EP 0230400, EP 0451216), einen single-chain-Antikörper etc. kodiert. Insbesondere kann ein solches Gen für eine humanisiertes Immunglobulinkette kodieren, die für variantes CD44 spezifisch ist (WO 95/33771). Zweckmäßig kann ein solches Nukleinsäuremolekül ein Expressionsvektor sein (Sambrook *et al.,* 16.3-16.29, 1989). Gegenstand der Erfindung sind besonders auch solche Expressionsvektoren, die nach der Einführung in eine eukaryontische Wirtszelle durch Rekombination in deren Genom integriert werden.

Ein weiterer Aspekt der vorliegenden Erfindung ist eine Wirtszelle, in die eines der genannten Nukleinsäuremoleküle eingeführt wurde. Bevorzugt ist in eine solche Wirtszelle ein Expressionsvektor eingeführt, der das Gen für ein heterologes Genprodukt in Verknüpfung mit dem Promotor des Ub/S27a-Gens und/oder weiteren regulatorischen Sequenzen enthält. Die erfindungsgemäße Wirtszelle ist bevorzugt eine Säugerzelle. Insbesondere kann die Wirtszelle eine Hamsterzelle, z.B. eine CHO- (CHO = Chinese hamster ovary; Urlaub *et* Chasin, 1980; vgl. auch Kaufman, 1987 sowie Referenzen darin, sowie Sambrook *et al.,* 16.28-16.29, 1989) BHK- (BHK = Baby hamster kidney) oder Hybridomzelle, besonders bevorzugt eine CHO-Zelle sein.

Ein weiterer Aspekt der vorliegenden Erfindung ist ein Verfahren zur Herstellung eines heterologen Genprodukts in einer eukaryontischen Wirtszelle, vorzugsweise einer Hamsterzelle, besonders bevorzugt einer CHO-Zelle, dadurch gekennzeichnet, daß eines der erwähnten Nukleinsäuremoleküle in die eukaryontische Wirtszelle eingeführt wird, die Wirtszelle kultiviert und das synthetisierte Genprodukt isoliert wird. Bei dem erfindungsgemäßen Verfahren wird das heterologe Genprodukt unter der Kontrolle von Promotorsequenzen und/oder regulatorischen Sequenzen des Ub/S27a-Gens, vorzugsweise aus Hamster, exprimiert. Vorteilhaft können für ein solches Verfahren Nukleinsäuremoleküle verwendet werden, die Promotorsequenzen und/oder regulatorische Sequenzen enthalten, die in der Abb. 5 enthalten sind. Besonders bevorzugt ist ein Verfahren, bei dem die Promotorsequenzen in der Sequenz enthalten sind, die den Positionen -161 bis -45 gemäß Abb. 5 entspricht. Auch hier liegt es im Können des Fachmanns, Teilsequenzen mit Promotoraktivität oder gleichwertige Varianten der offenbarten Sequenzen herzustellen.

Ein starker homologer Promotor des Hamsters, der für die Herstellung heterologer Genprodukte in Hamsterzellen, insbesondere CHO-Zellen sehr vorteilhaft ist, wird hiermit erstmalig bereitgestellt. Insbesondere betrifft die Erfindung einen starken homologen Promotor des Hamsters, der in CHO-Zellen im CAT-Assay gemäß Beispiel 4 eine größere Aktivität aufweist als der Thymidinkinasepromotor aus *Herpes simplex.* Vorteilhaft weist ein solcher Promotor eine Transkriptionsaktivität auf, die mindestens in der gleichen Größenordnung liegt wie diejenige des SV40-Promotors. In der gleichen Größenordnung soll hier bedeuten, daß der erfindungsgemäße Promotor mindestens 50%, besser mindestens 80%, noch mehr bevorzugt mindestens 90% der Aktivität des SV40-Promotors im CAT-Assay gemäß Beispiel 4 hat. Bevorzugt ist ein solcher Promotor dadurch gekennzeichnet, daß er mindestens eines der Merkmale: GC-reicher Sequenzbereich, Spl-Bindungsstelle, Polypyrimidinelement, Abwesenheit einer TATA-Box aufweist. Besonders bevorzugt ist ein solcher Promotor, der eine Sp1-Bindungsstelle bei Abwesenheit einer TATA-Box aufweist. Ferner ist ein solcher Promotor bevorzugt, der konstitutiv aktiviert ist und insbesondere unter serumhaltigen, serumarmen und serumfreien Zellkulturbedingungen gleichermaßen aktiv ist. In einer anderen Ausführungsform handelt es sich um einen induzierbaren Promoter, insbesondere um einen Promotor, der durch Serumentzug aktiviert wird. Eine besonders vorteilhafte Ausführungsform ist ein Promotor mit einer Sequenz, die in Abb. 5 enthalten ist. Besonders bevorzugt ist dabei eine Sequenz, die in der Sequenz enthalten ist, die den Positionen -161 bis -45 gemäß Abb. 5 entspricht.

Ein weiterer Aspekt der vorliegenden Erfindung ist ein Verfahren zur Expression eines heterologen Genprodukts in Hamsterzellen im Umfang der Patentansprüche, vorzugsweise CHO-Zellen, das dadurch gekennzeichnet ist, daß das Genprodukt unter der Kontrolle eines starken homologen Promotors des Hamsters exprimiert wird. In bevorzugten Ausführungsformen ist ein solcher Promotor durch Merkmale gekennzeichnet, wie sie im vorigen Absatz beschrieben wurden.

Ein weiterer Aspekt der vorliegenden Erfindung ist die Verwendung eines Promotors, wie er vorstehend beschrieben wurde, zur Herstellung eines heterologen Genprodukts in Hamsterzellen, vorzugsweise CHO- oder BHK-Zellen.

Die beschriebenen Promotorsequenzen können in einer Expressionskassette in funktionellen Zusammenhang mit weiteren regulatorischen Sequenzen gebracht werden. Beispielsweise können sie mit Enhancer-Sequenzen funktionell verknüpft und so die Transkriptionsaktivität gesteigert werden. Dabei kann es sich um einen oder mehrere Enhancer und/oder mehrere Kopien einer Enhancer-Sequenz handeln. Beispielsweise kann dabei ein CMV- oder ein SV40-Enhancer verwendet werden. Der humane CMV-Enhancer gehört dabei zu den stärksten bisher identifizierten Enhancem. Ein Beispiel für einen induzierbaren Enhancer ist der Metallothionein-Enhancer, der durch Glukokortikoide oder Schwermetalle stimuliert werden kann. Eine weitere mögliche Modifikation wäre z.B. die Einführung multipler Spl-Bindungsstellen. Die Promotorsequenzen können ferner mit regulatorischen Sequenzen kombiniert werden, die eine Steuerung/Regulierung der Transkriptionsaktivität gestatten. So kann der Promotor reprimierbar/induzierbar gemacht werden. Dies kann beispielsweise durch die Verknüpfung mit Sequenzen geschehen, die Bindungsstellen für positiv oder negativ regulierende Transkriptionsfaktoren darstellen. Der oben genannte Transkriptionsfaktor SP-1 beispielsweise hat einen positiven Einfluß auf die Transkriptionsaktivität. Ein weiteres Beispiel ist die Bindungsstelle für das Aktivatorprotein AP-1, das sowohl in positiver als auch in negativer Weise auf die Transkription einwirken kann. Die Aktivität des AP-1 kann durch verschiedenste Faktoren, wie z.B. Wachstumsfaktoren, Zytokine und Serum, gesteuert werden (Faisst *et* Meyer, 1992, und Referenzen darin). Die Transkriptionseffizienz kann auch dadurch gesteigert werden, daß die Promotorsequenz durch Mutation (Substitution, Insertion, Deletion) von einer, zwei, drei oder mehr Basen verändert wird und dann im CAT-Test gemäß Beispiel 4 gemessen wird, ob sich dadurch die Promotoraktivität erhöht. Durch die in diesem Absatz beschriebenen Maßnahmen kann eine optimierte Expressionskassette erhalten werden, die von hohem Nutzen fur die Expression heterologer Genprodukte, insbesondere in CHO-Zellen, ist. Eine durch eine oder mehrere solcher Maßnahmen erhaltene Expressionskassette ist daher ebenfalls Gegenstand der Erfindung

In DNaseI-Footprint- und Mutationsanalysen kann untersucht werden, welche Faktoren die Expression beeinflussen und ob die Promotoraktivität durch die Deletierung von möglicherweise vorhandenen negativen Kontrollelementen und durch die Einführung von weiteren positiven Kontrollelementen noch gesteigert werden kann. Untersuchungen anderer Arbeitsgruppen haben außerdem gezeigt, daß die Expression des Ub/S27a-Genes ganz offensichtlich durch verschiedene Faktoren reguliert werden kann. So zeigte die Gruppe um Shimbara, daß bei einer terminalen *in vitro* Differenzierung von humanen Leukämie-Zellinien (HL-60, K562, U937, THO1) durch Zugabe von verschiedenen Substanzen wie TPA (12-O-Tetra-decanoylphorphol-13-acetat), DMSO, Retinsäure und 1,25-Dihydroxyvitamin D3 in das Kulturmedium die Expression des Ub/S27a-Genes herunterreguliert wird (Shimbara et al.; 1993). Und die Gruppe um Wong stellte eine Überexpression des Ub/S27a-Gens in Carcinomazellen des Enddarms fest (Wong et al., 1993). Die Genexpression korrelierte dabei mit den klinischen Tumorstadien mit einer höheren Expression in weiter fortgeschrittenem Krebs.

Die Ausführung der Erfindung ist dem Fachmann in Kenntnis des Offenbarungsgehaltes dieser Anmeldung mit an sich bekannten Methoden sowie gemäß der Beispiele möglich.

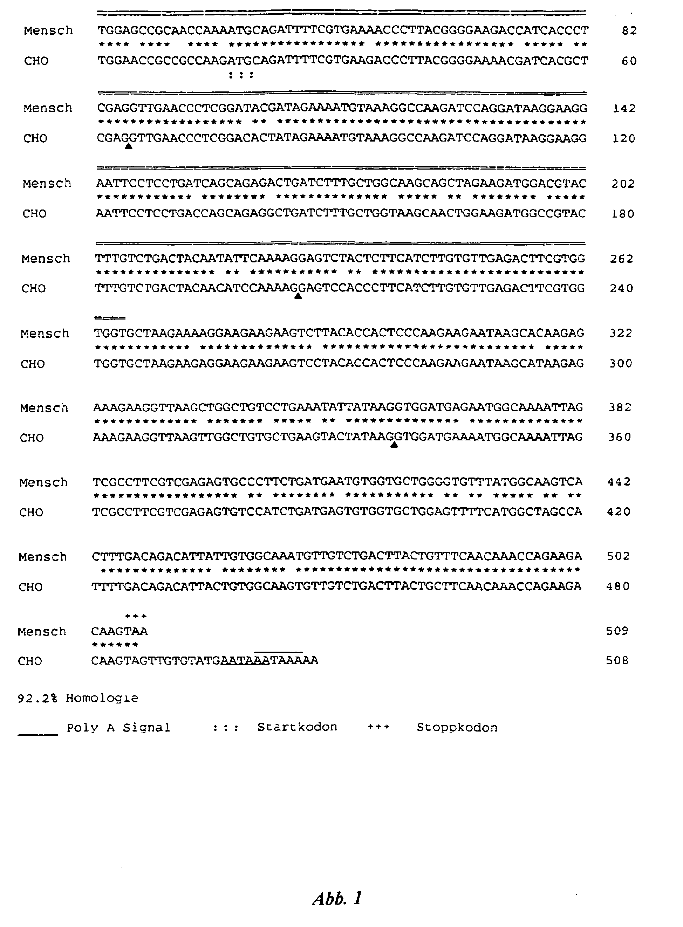

Das komplette Ub/S27a-Gen, die 5'-untranslatierte Region des Ub/S27a-Gens bzw. ausgewählte Fragmente davon können in Kenntnis der Sequenzen gemäß Abb. 1, 2 und 5 mit verschiedenen Standardmethoden erhalten werden. Aus der Sequenz gemäß Abb. 5 kann beispielsweise ein geeigneter Abschnitt ausgewählt und eine Oligonukleotid-Sonde chemisch synthetisiert werden, die die Sequenz dieses Abschnitts hat (Sambrook *et al.,* 11.3-11.44, 1989). Mit einer solchen Sonde kann durch Hybridisierung aus einer genomischen Bibliothek (Sambrook *et al.,* 9 4-9.62, 11.45-11.61, 1989) des Hamsters das Ub/S27a-Gen bzw. dessen 5'-untranslatierte Region kloniert werden. Die 5'-untranslatierte Region bzw. spezielle Fragmente davon können auch leicht durch PCR-Amplifikation mit entsprechenden Primern aus einer genomischen Bibliothek erhalten werden (Sambrook *et al.,* 14.5-14.35, 1989). Diese Methode eignet sich besonders zur Herstellung ausgewählter Fragmente der Promotorregion, etwa des Bereichs von -161 bis -45 oder eines Abschnitts dieses Bereiches. Fragmente der 5'- untranslatierten Region wie etwa die in Tabelle 1 aufgeführten können auch durch limitierten Exonuklease III-Verdau aus größeren DNA-Fragmenten erhalten werden (Sambrook *et al.,* 15.14-15.19; 5.84-5.85, 1989). Solche DNA-Moleküle können auch chemisch synthetisiert oder aus chemisch synthetisierten Fragmenten durch Ligation erzeugt werden. Das Ub/S27a-Gen einer anderen Species, vorzugsweise Säugerspecies, bzw. dessen 5'-untranslatierte Region kann durch Kreuzhybridisierung mit Sonden aus dem 5'-untranslatierten Bereich des Hamster-Ub/S27a-Gens oder etwa Sonden aus dem S27a-Anteil des Hanster-Ub/S27a-Gens isoliert werden.

Es steht im Stand der Technik eine Anzahl von Expressionsvektoren zur Verfügung die im Zusammenhang mit der vorliegenden Erfindung verwendet werden können. Die erfindungsgemäßen Promotor- und/oder regulatorischen Sequenzen können an Stelle der in diesen Vektoren vorhandenen Promotorelemente integriert werden und so die Expression des jeweiligen Gens steuern, das mit diesem Vektor exprimiert werden soll. Kommerziell erhältliche Vektoren, die sich zur Integration des erfindungsgemäßen Promotors gut eignen, sind beispielsweise der pCAT-Basic Vector (Promega; kompilierte Sequenz zugänglich über EMBL Accession No. X65322) oder der pCAT-Enhancer Vector, der zusätzlich noch einen SV40-Enhancer enthält (Promega; kompilierte Sequenz zugänglich über EMBL Accession No. X65319). Ein Beispiel für einen promotorlosen Vektor ist das Plasmid pBL-CAT6 (Boshart *et al.,* 1992; siehe auch Luckow et Schütz, 1987). Die erfindungsgemäßen Promotorsequenzen können in die HindIII-, SphI-, PstI-, SalI-, XbaI-, BamHI-, BgIII- oder XhoI-Restriktionsschnittstellen dieses Vektors ligiert werden und so mit dem in diesem Vektor enthaltenen Chloramphenicol-Transferase (CAT)-Gen funktionell verknüpft werden. Anstelle des CAT-Gens kann auch ein anderes Gen, etwa fur Gewebsplasminogenaktivator, in diesen Vektor integriert werden. Das CAT-Gen kann z.B. durch einen Doppelverdau mit den Restriktionsenzymen XhoI und ClaI aus dem Vektor pBL-CAT6 entfernt werden. Die dabei deletierte SV40 3' untranslatierte Region, die die Intronsequenz und das Polyadenylierungssignal enthält, kann ggf. (d.h. sofern deren Funktion nicht durch die geneigenen 3'-Sequenzen übernommen wird) nachfolgend wieder eingebaut werden, indem man z.B. diese SV40-Region mittels PCR amplifiziert und dabei gleich mit geeigneten Restriktionsschnittstellen an beiden Enden versieht, damit nachfolgende Umklonierungen, z.B. bei Einführung eines anderen gewünschten Genes, erleichtert werden. Eine andere Strategie der Auswechslung des CAT-Genes gegen ein anderes Gen ist, daß man zunächst mittels PCR und geeigneter mutagener Oligonukleotide eine singuläre Restriktionsschnittstelle am 5'-Ende der SV40-Region in den Vektor pBL-CAT6 einführt, die es nachfolgend ermöglicht, gezielt das CAT-Gen zu entfernen. Eine weitere Möglichkeit besteht in der Verwendung des Vektors pLUC, der im Prinzip wie pBL-CAT6 aufgebaut ist, aber anstelle des CAT-Gens ein Luciferase-Reportergen enthält. Dieses läßt sich leicht durch einen XhoI/EcoNI-Doppelverdau entfernen, wobei die SV40-Sequenz im Vektor verbleibt.

Zur Erzeugung stabiler Zellinien mit hoher Expression des heterologen Gens wählt man vorteilhaft einen Vektor, der die Selektion von Transformanten gestattet, die den Vektor in ihr Chromosom integriert und das integrierte heterologe Gen amplifiziert haben. Vektoren für solche Selektions-/Amplifikationsverfahren wie das DHFR/Methotrexat-Verfahren in DHFR-defizienten Zellinien (z.B CHO-DUKX) sind ebenfalls Stand der Technik (Sambrook *et al.,* 16.9-16.15; 16.28-16.29, 1989).

Verfahren zur Einbringung der erhaltenen Vektoren in Wirtszellen ebenso wie die Selektion und Kultur der Transformanten kann der Fachmann Standardwerken entnehmen (z.B. Sambrook *et al.,* 16.3-16.81, 1989)

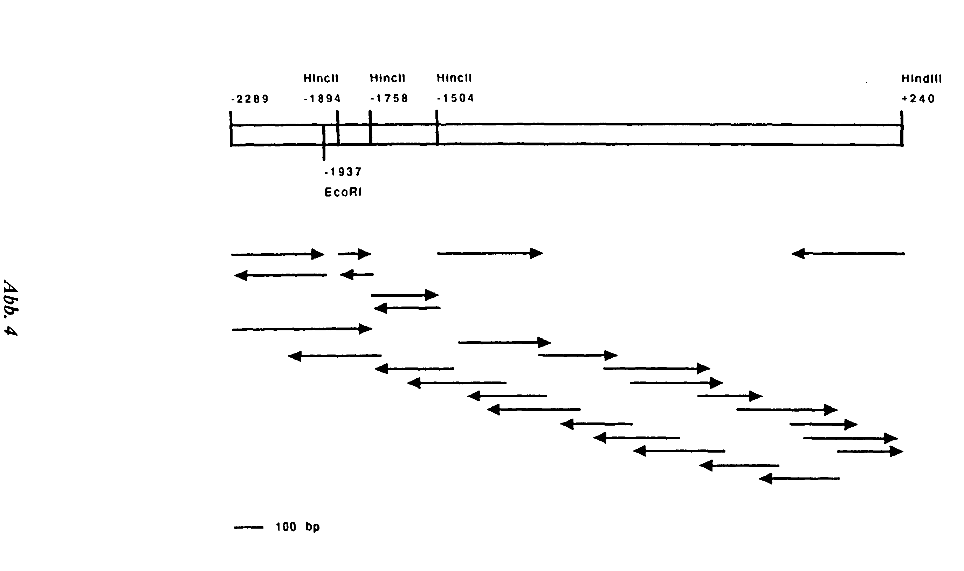

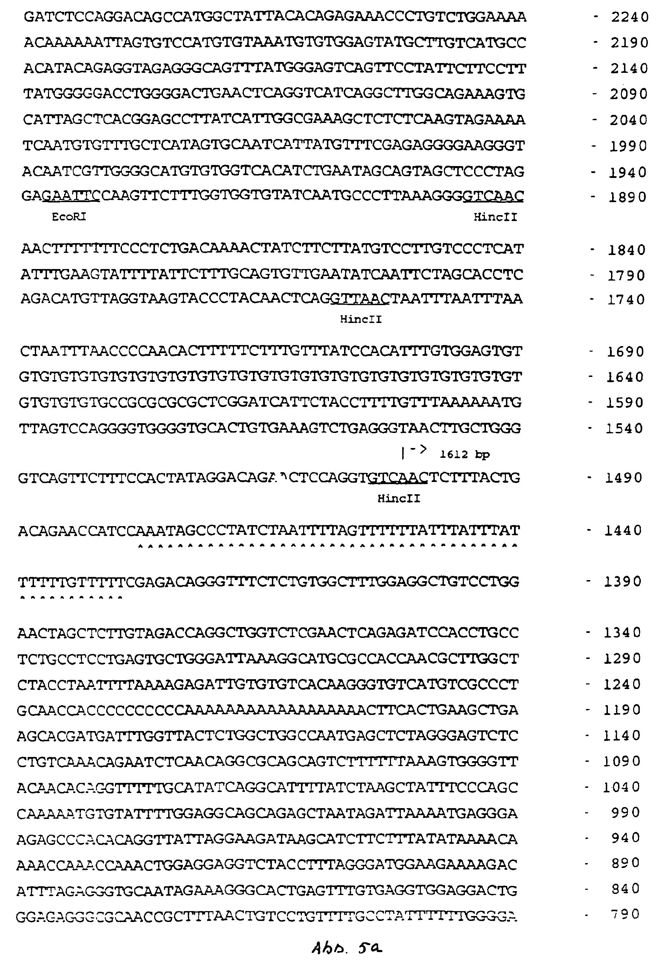

Abgesehen von der Optimierung des Promotors bietet sich noch ein ganz anderer Ansatzpunkt zur Verbesserung der Produktausbeute, und zwar über einen Gendosiseffekt. Mit steigender Kopienzahl der ins Genom integrierten Vektorkonstrukte sollte auch die Menge an produzierten Transkripten zunehmen. Eine spontane Amplifikation der eingeführten Konstrukte, die in einer stabilen Integration einer Vielzahl von Kopien resultiert, kann durch die Verwendung einer Sequenz mit amplifikationsfördernden Eigenschaften, sogenannten "amplification promoting sequences", erzielt werden. Eine solche Sequenz, die einen sehr hohen AT-Gehalt besitzt, war z.B. aus der nicht-transkribierten Intergenregion der ribosomalen Mausgene isoliert worden (Wegner et al., 1989) und wurde bereits erfolgreich zur stabilen und effizienten Inhibierung der HIV-1 Replikation durch antisense RNA eingesetzt (Meyer et al., 1993). Ein 49 bp großer Sequenzbereich der Ub/S27a 5' untranslatierten Region (Position - 1477 bis - 1429; Abb.4) mit einem hohen AT-Gehalt von 88% weist große Homologie zu den oben beschriebenen amplifikationsfördernden Sequenzen der Maus auf. Daß dieser CHO-Sequenzbereich ebenfalls solche Eigenschaften besitzt, kann durch den Einsatz von Ub/S27a-Promotorkonstrukten, denen diese CHO-Sequenz vorgeschaltet ist, überprüft werden.

### Beschreibung der Abbildungen

***Abb.1: Vergleich der DNA-Sequenz des kompletten Ubiquitin*/*S27a cDNA-Klons aus CHO-Zellen mit der humanen cDNA-Sequenz*** Vergleich der CHO Ub/S27a cDNA-Sequenz mit der humanen cDNA-Sequenz (Adams et al., 1992). Identitäten innerhalb der Sequenz sind durch "*" hervorgehoben. Der Ubiquitinanteil ist durch Doppellinien über der Sequenz gekennzeichnet. Die Positionen der drei Introns sind durch Dreiecke markiert. Poly A Signal ::: Startkodon +++ Stoppkodon

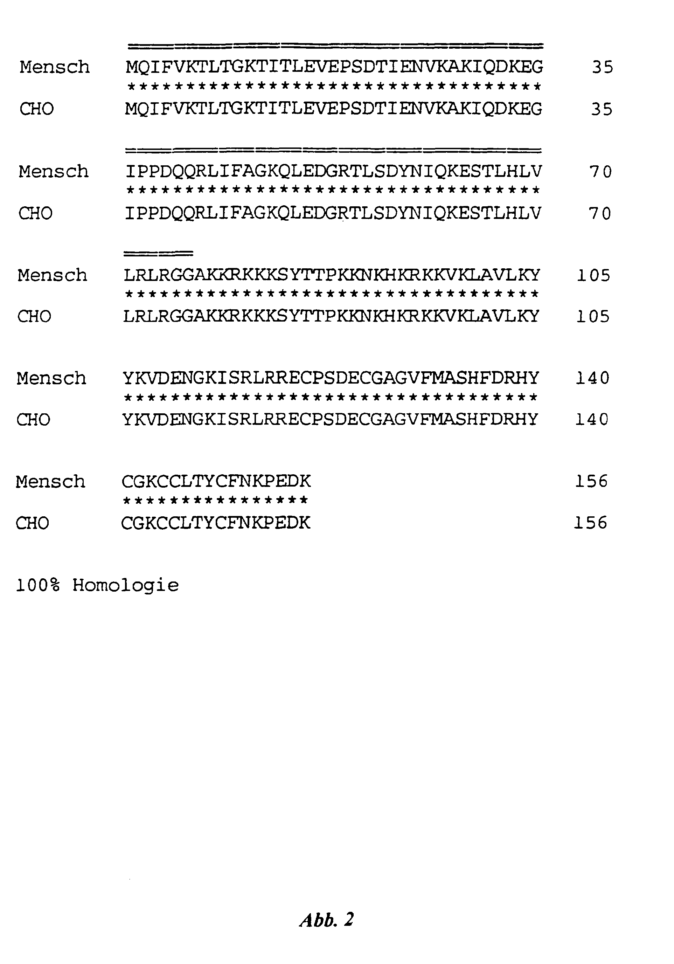

***Abb.2: Aminosäuresequenz** des* ***Ubiquüinjusionsproteins Ub*/*S27a.*** Vergleich der aus der CHO cDNA -Sequenz abgeleiteten Ub/S27a-Aminosäuresequenz mit der humanen Aminosauresequenz (Adams et al., 1992) Identitäten innerhalb der Sequenzen sind durch "*" gekennzeichnet. Die Ubiquitineinheit von 76 Aminosäuren ist durch Doppellinien über der Sequenz hervorgehoben.

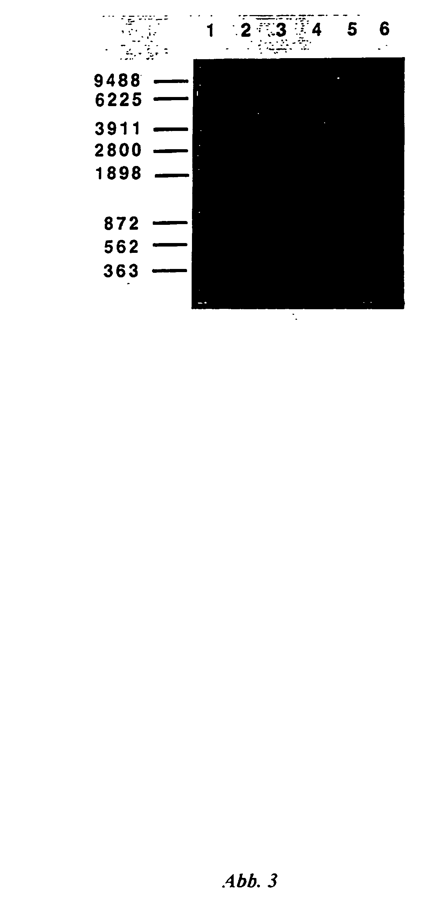

***Abb.3: Analyse der Ub*/*S27a-Transkriptlevel in CHO-Zellen.*** Die denaturierte zytoplasmatische Gesamt-RNA (10 µg) von serumkultivierten und serumfrei kultivierten CHO-Zellen wurde in einem Formaldehyd-haltigen Agarosegel elektrophoretisch aufgetrennt und auf eine Nylonmembran transferiert. Als Hybridisierungsprobe wurde die ³²P-markierte Ub/S27a-cDNA (508 bp) aus CHO eingesetzt. Die Expositionszeit des Röntgenfilms betrug 3 Stunden.
1 + 3 serumkultivierte CHO-Zellen
2 + 4 serumfrei kultivierte CHO-Zellen
5 serumkultivierte CHO-Zellen wurden für 24 Stunden in serumfreiem Medium kultiviert
6 serumfrei kultivierte CHO-Zellen wurden für 24 Stunden in serumhaltigem Medium kultiviert

***Abb. 4: Strategie zur Sequenzierung der Ub*/*****S27a 5' untranslatierten Region.*** Die 2.5 kb große 5' untranslatierte Region des Ub/S27a-Gens ist schematisch dargestellt. Es wurden sowohl subklonierte Restriktionsfragmente als auch durch Exonuklease III-Verdau erzeugte Deletionsklone sequenziert, wobei Ausmaß und Richtung der Sequenzierungen durch Pfeile angezeigt sind.

***Abb. 5: Genomische DNA-Sequenz der Ub*/*S27a 5' untranslatierten Region***. Zeichenerklärung:
- ____: Restriktionsschnittstellen
- | - >: 5' Ende der Promotordeletionsklone
- *: 3' Ende der Promotordeletionsklone
- ^^^^^^: Homologie zu amplifikationsfördernden Sequenzen
- " " " " " ": polypyrimidinreicher Sequenzbereich
- :::: Startkodon
- +1, +13: durch S1 Nukleasekartierung bestimmte Transkriptionsstartstellen
- + 85: über Primer-Extension erhaltenes 5' Ende
- > > > > > >: Spl-Bindungsstelle

Die Nukleotide sind in Relation zur Transkriptionsstartstetle, die mit +1 bezeichnet ist, numeriert Die Restriktionsschnittstellen für SacII und Eagl, die für GC-reiche Sequenzbereiche spezifisch sind, sowie die zu Subklonierungszwecken herangezogenen Restriktionsschnittstellen für EcoRI, HincII und HindIII sind in der Sequenz durch Unterstreichung hervorgehoben. Zur Kennzeichnung der Intronsequenz wurden Kleinbuchstaben verwendet. Die Aminosäuresequenz ist unter der DNA-Sequenz wiedergegeben.

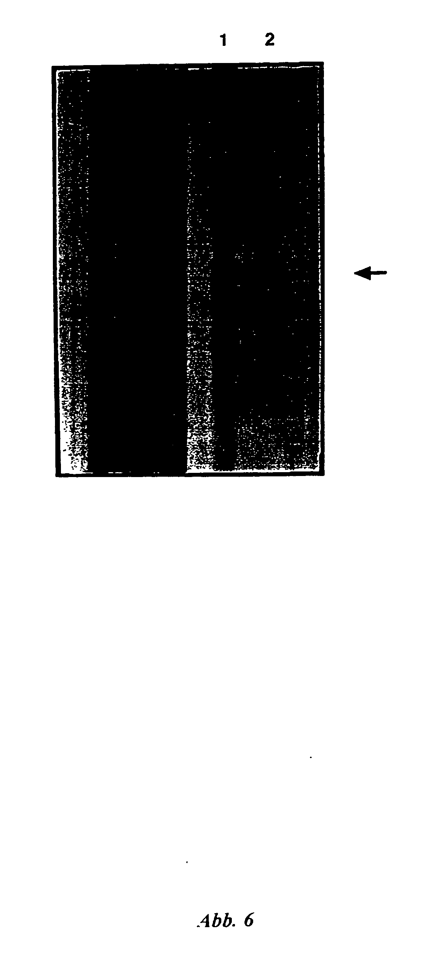

***Abb.6: Bestimmung des Transkriptionsstartpunktes durch Primer-Extension.*** Die Primer-Extensionsanalyse wurde mit 5 µg zytoplasmatischer Gesamt-RNA von serumkultivierten CHO-Zellen durchgeführt (Bahn 1). Als Kontrolle wurde 5 µg Hefe tRNA verwendet (Bahn 2). Der eingesetzte ³²P-endmarkierte Primer hybridisierte mit dem S27a-Anteil der Ub/S27a mRNA (Nukleotide + 256 bis + 276 in der cDNA-Sequenz). Die Extensionsprodukte wurden in einem 6%igen denaturierendem Polyacrylamidgel aufgetrennt. Als Größenmarker wurde eine Sequenzierungsreaktion benutzt. Die Länge des Extensionsproduktes, dessen Position durch einen Pfeil gekennzeichnet ist, betrug 304 Nukleotide.

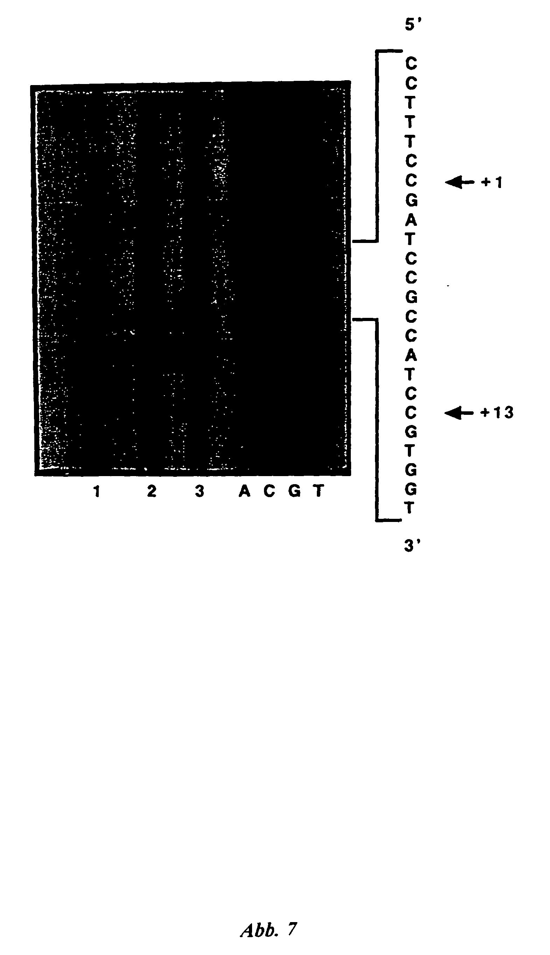

***Abb. 7: Ermittlung des Transkriptionsstartpunktes durch S1 Nuklease-Kartierung.*** Eine der Gegenstrangsequenz entsprechende ³²P-endmarkierte Einzelstrangprobe, die den gesamten Bereich der 5' untranslatierten Region (- 2289 bis + 176) umfaßte, wurde mit 5 µg Gesamt-RNA (Bahn 2) bzw. zytoplasmatischer Gesamt-RNA (Bahn 3) aus serumkultivierten CHO-Zellen hybridisiert. Als Kontrolle wurde 5 µg Hefe tRNA eingesetzt (Bahn 1). Die vor dem S1 Nuklease-Abbau geschützten Fragmente wurden in einem 8%igen denaturierendem Polyacrylamidgel aufgetrennt. Als Größenmarker diente eine Sequenzierungsreaktion, in der der zur eingesetzten Hybridisierungsprobe komplementäre DNA-Strang sequenziert wurde. Der zur Sequenzierung benutzte Primer hybridisierte dabei mit der Nukleotidsequenz von + 157 bis + 176. Die Positionen der abbaugeschützten DNA-Fragmente sind in der hervorgehobenen Sequenz durch Pfeile markiert. Dem distalen Transkriptionsstartpunkt wurde die Nukleotidposition +1 zugeordnet.

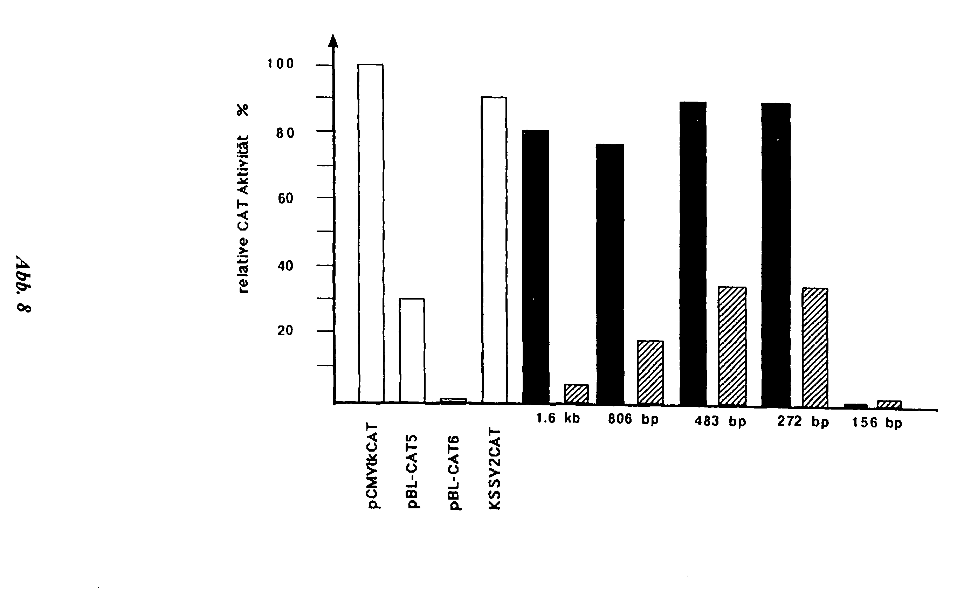

***Abb.8: Funktionelle Analyse der Ub*/*S27a-Promotoraktivität*.** Vektorkonstrukte, in denen serielle Deletionen der 5' flankierenden Region des Ub/S27a-Gens in beiden Orientierungen mit dem CAT-Reportergen fusioniert worden waren, wurden zur transienten Transfektion von serumkultivierten CHO-Zellen eingesetzt. Als Kontrolle wurden Plasmide eingesetzt, in denen das CAT-Reportergen unter der Kontrolle eines viralen Promotors stand. Insgesamt wurden vier voneinander unabhängige Transfektionsexperimente durchgeführt.

Die relative CAT-Aktivität der verschiedenen Vektorkonstrukte ist als Prozent der CAT-Aktivität in pCMVtkCAT transfizierten CHO-Zellen, die als 100% angenommen wurde, angegeben und repräsentiert den Mittelwert (Standardabweichung in allen Fällen ≤ 5%) aus den vier Transfektionsexperimenten. Sämtliche CAT-Aktivitaten wurden bezüglich eingesetzter Proteinmenge sowie Transfektionseffizienz, die durch Messung der β-Galactosidase-Aktivität des co-transfizierten Kontrollplasmides pCMVtklacZ bestimmt wurde, korrigiert.
□ Kontrollplasmide: ohne Promotor oder virale Promotoren (tk, SV40)
■ Ub/S27a 5' untranslatierte Region: 5'-3' Orientierung in pBL-CAT6
▨ Ub/S27a 5' untranslatierte Region: 3'-5' Orientierung in pBL-CAT6

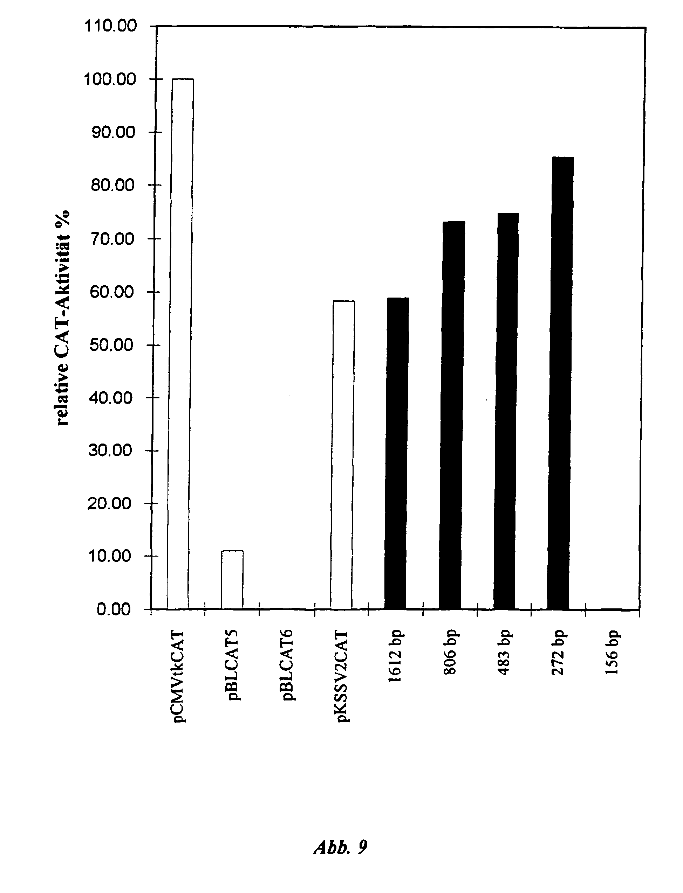

***Abb. 9: Funktionelle Analyse der Aktivität des Ub*/*S27a-Promotors in transient transfizierten BHK-Zellen (BHK21).***
□ Kontrollplasmide: ohne Promotor oder virale Promotoren (tk, SV40)
■ Ub/S27a 5' untranslatierte Region: 5'-3' Orientierung in pBL-CAT6

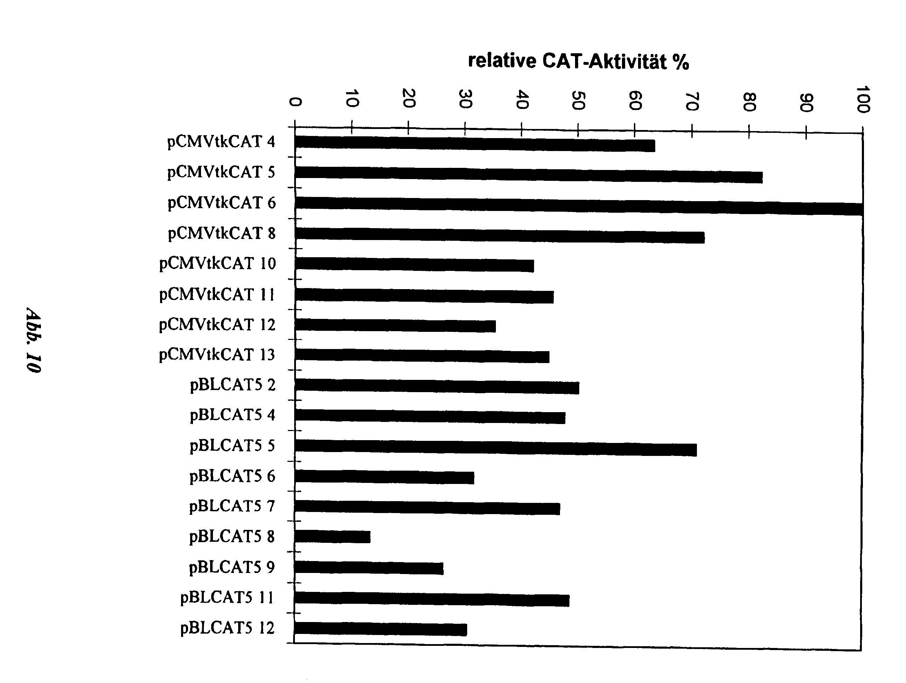

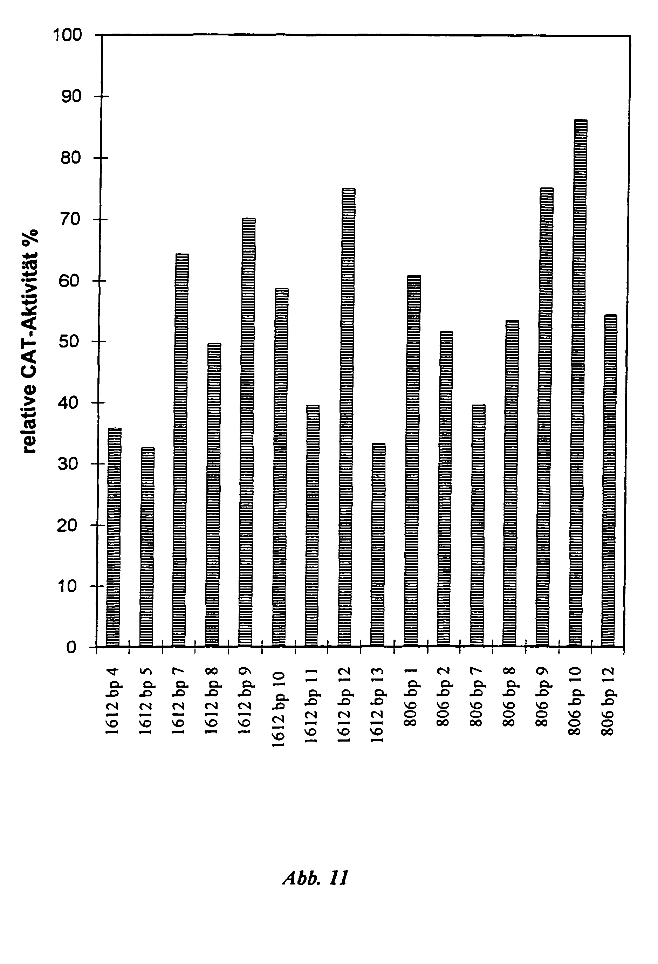

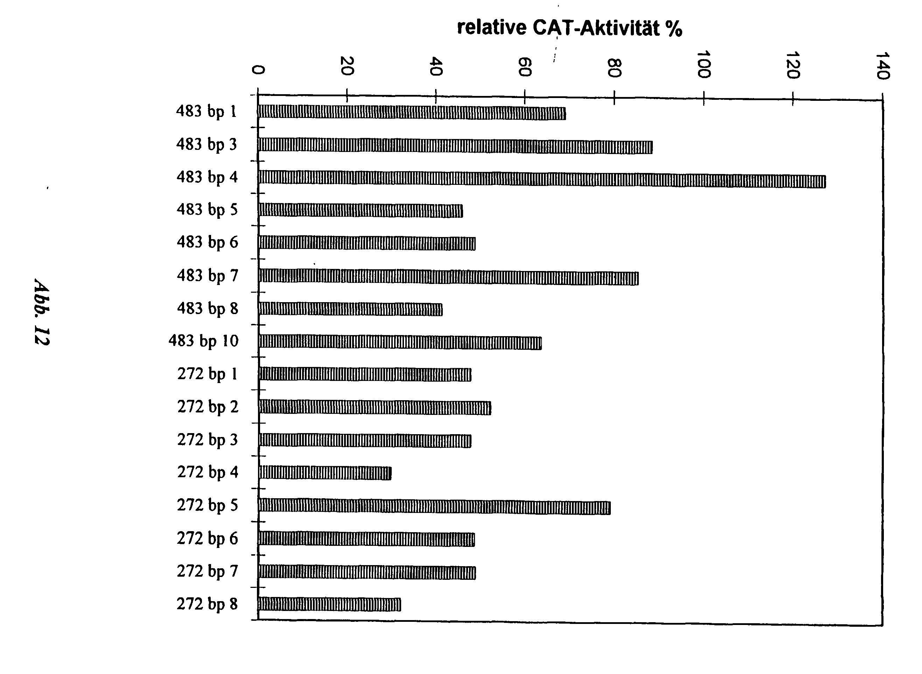

***Abb. 10: Funktionelle Analyse der Aktivität des Ub*/*S27a-Promotors in stabil transfizierten CHO-Zellen (I).*** Dargestellt ist die CAT-Aktivität in Zellen, die mit dem Vektor pCMVtkCAT (tk-Promotor/CMV-Enhancer) oder dem Vektor pBL-CAT5 (tk-Promotor) stabil transfiziert wurden (vgl. Beispiele/Plasmide, Beispiel 6). Gezeigt sind jeweils mehrere verschiedene Klone. Die CAT-Aktivität ist auf die Aktivität des Klons pCMVtkCAT 6, der die höchste Aktivität hatte, normiert (=100%). Die Graphik dient dem Vergleich mit den Abbildungen 11 und 12.

***Abb. 11: Funktionelle Analyse der Aktivität des Ub*/*S27a-Promotors in stabil transfizierten CHO-Zellen (II)***. Gezeigt ist die CAT-Aktivität verschiedener Vektorkonstrukte, die als Prozent der CAT-Aktivität des Klons 6 der mit pCMVtkCAT transfizierten CHO-Zellen aus Abb. 10 dargestellt ist. Fragmente aus der Promotorregion gemäß Tabelle 1 wurden in pBL-CAT6 in 5' → 3'-Orientierung integriert. Die Abszisse gibt an, welches Fragment verwendet wurde. Gezeigt ist die Aktivität von jeweils mehreren verschiedenen Klonen.

***Abb. 12: Funktionelle Analyse der Aktivität des Ub*/*****S27a-Promotors in stabil transfizierten CHO-Zellen (lll).*** Gezeigt ist die CAT-Aktivität verschiedener Vektorkonstrukte, die als Prozent der CAT-Aktivität des Klons 6 der mit pCMVtkCAT transfizierten CHO-Zellen aus Abb. 10 dargestellt ist Fragmente aus der Promotorregion gemäß Tabelle 1 wurden in pBL-CAT6 in 5' → 3'-Orientierung integriert. Die Abszisse gibt an, welches Fragment verwendet wurde. Gezeigt ist die Aktivität von jeweils mehreren verschiedenen Klonen.

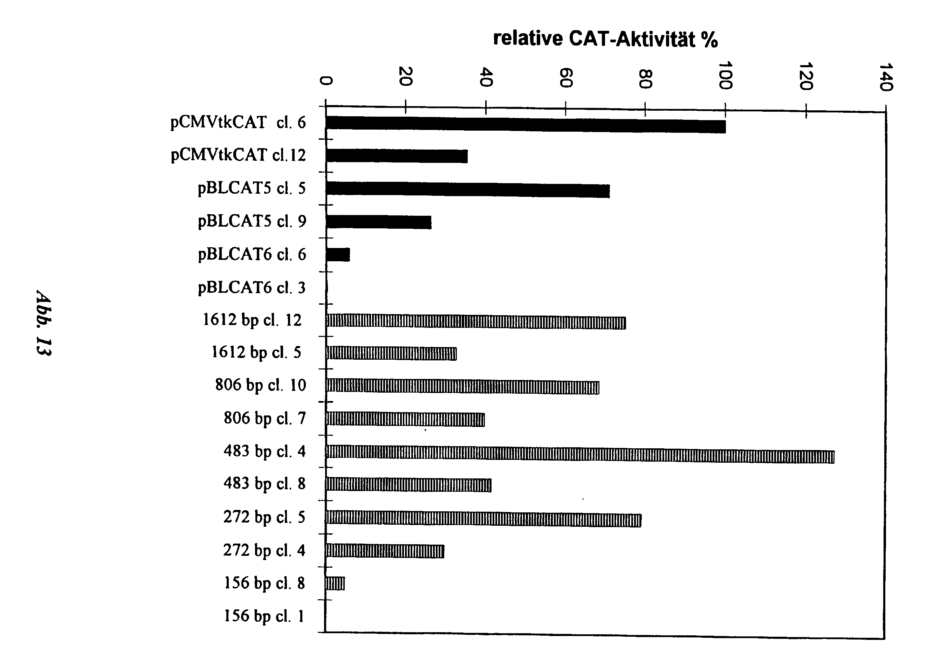

***Abb. 13: Funktionelle Analyse der Aktivität des Ub*/*S27a-Promotors in stabil transfizierten CHO-Zellen (IV).*** Diese Abbildung stellt eine Zusammenfassung der Daten der Abbildungen 10 bis 12 dar. Gezeigt sind die Zellklone mit der höchsten und der niedrigsten CAT-Expression für jedes Vektorkonstrukt, das in diesem Experiment verwendet wurde (nur Plasmide mit 5' → 3'-Orientierung des Ub/S27a-Promotors).

### Beispiele

### Plasmide

Für die Durchführung sämtlicher Genmanipulationen wurde der Vektor pBluescript SK- verwendet (Stratagene Cloning Systems, Stratagene GmbH, Heidelberg, Deutschland). Zur Expressionsanalyse wurden eukaryontische Expressionsvektoren eingesetzt, die das als Reportergen dienende bakterielle Chloramphenicol-Acetyltransferase (CAT)-Gen enthielten. Das CAT-Gen ist dabei mit verschiedenen viralen Promotoren fusioniert. In pBL-CAT5 ist es der Thymidinkinasepromotor (tk) des Herpes simplex Virus (Boshart et al., 1992; kompilierte Sequenz zugänglich über GenBank Accession No. M80483), in pCMVtkCAT der tk-Promotor in Kombination mit einem Enhancer des humanen Cytomegalievirus (CMV) (Christoph Winkler, Würzburg) und in pKSSV2CAT der SV40-Promotor (Tsonis et al., 1988) pBL-CAT6 diente als Negativkontrolle und enthält ein promotorloses CAT-Gen (Boshart et al., 1992; kompilierte Sequenz zugänglich über GenBank Accession No. M80484). Im Vektor pCMVtklacZ steht die Expression des bakteriellen β-Galactosidase-Gens unter der Kontrolle der CMV-Enhancer/tk-Promotorkombination (Christoph Winkler, Würzburg).

### Zellkultur

CHO-DUKX Zellen (Urlaub und Chasin, 1980) wurden in Dulbecco's Modified Eagle's Medium/ Ham's F12 Medium (1:1), supplementiert mit 5% fötalem Kälberserum, in Roux-Flaschen in einem CO₂ begasbaren Brutschrank bei 90% Luftfeuchtigkeit, 37°C und 5% CO₂ kultiviert. Für die serumfreie Kultivierung wurden CHO-DUKX Zellen verwendet, die langsam an ein Wachstum in Medium ohne Serum adaptiert worden waren und nun permanent in serumfreiem Medium kultiviert werden. Diese Zellen wurden als Suspensionskulturen in Spinnerflaschen in Iscove's Modified Dulbecco's Medium/Ham's F12 Medium (1:1) kultiviert, das mit niedermolekularem Pepton (Aldag, Hamburg), Insulin und Transferrin (Canada Packars) supplementiert wurde.

### Beispiel 1: Differentielle Hybridisierung rekombinanter CHO cDNA Genbanken

Unter Verwendung von polyadenylierter mRNA aus CHO-Zellen, die entweder in Serum oder ohne Serum kultiviert worden waren, wurden zunächst zwei cDNA Genbanken in λZAPII, bestehend aus 4.2 x 10⁶ bzw. 2.9 x 10⁵ rekombinanten Phagenklonen, hergestellt.

Die zytoplasmatische RNA wurde von NP40-lysierten Zellen präpariert und durch Phenol/Chloroform-Extraktionen gereinigt (Nicolaides und Stoeckert, 1990). Polyadenylierte mRNA von CHO-Zellen, die mit oder ohne Serum kultiviert worden waren, wurde durch Affinitätschromatographie an einer Oligo(dT)-Cellulosesäule gewonnen (Sambrook et al., 1989). Zur Herstellung der cDNA wurde das cDNA Synthesekit von Pharmacia LKB verwendet, wobei zur Erststrangsynthese ein Oligo (dT)-Primer eingesetzt wurde. Die cDNA wurde in den EcoRI-verdauten Vektor λZAPII kloniert (Strategene Cloning Systems). Filterduplikate der nicht-amplifizierten CHO cDNA Genbank aus serumfrei kultivierten CHO-Zellen wurden durch differentielle Hybridisierung gescreent. Als Probe wurde GesamtcDNA von serumkultivierten und serumfrei kultivierten CHO-Zellen eingesetzt, die durch "random priming" (Prime a Gene labelling Kit; Promega Cooperation, Promega Biotec, Madison, WI, USA) mit (α-³²P) dCTP (6000 Ci/mmol; Amersham International plc, Amersham-Buchler, Braunschweig, Deutschland) markiert worden war. Die Hybridisierung wurde wie in der Northern Blot Analyse (s.u., Beispiel 2) beschrieben durchgeführt. Phagenplaques, die mit beiden cDNA-Proben eine starke Hybridisierung zeigten, wurden isoliert und nochmals einer differentiellen Hybridisierung unterzogen. Aus den rekombinanten λZAPII Phagen wurden Phagemide durch *in vivo* Exzision unter Einsatz des Helferphagen R408 gewonnen (λZAPII cloning kit-Protokoll; Stratagene Cloning Systems).

Ungefähr 6000 Phagenklone wurden so gescreent. Insgesamt wurden 12 rekombinante Phagenklone isoliert, die eine besonders starke Hybridisierung mit beiden Gesamt-cDNA-Proben zeigten.

DNA-Sequenzen wurden nach der Didesoxymethode unter Verwendung des T7 Sequenzierungskits .von Pharmacia LKB ermittelt. Als Primer wurden sowohl T3 und T7 Promotorprimer als auch genspezifische Primer eingesetzt. In allen Fällen wurden die DNA-Proben mit [α-³⁵S] dATP bzw. [α-³⁵S] dCTP (10 µCi; Amersham International plc) markiert. Die Reaktionsprodukte wurden in einem 6%igen Polyacrylamid-Sequenzierungsgel elektrophoretisch aufgetrennt. Zur Sequenzanalyse wurden die Datenbanken von GenBank und EMBL herangezogen.

Einer der isolierten cDNA-Klone kodierte für ein Fusionsprotein, Ub/S27a, bestehend aus einer Ubiquitin-Monomereinheit und einem ribosomalen Protein der kleinen Untereinheit, S27a (Abb.1). Die größte Homologie besteht mit der humanen Ub/S27a-Sequenz (Adams et al., 1992) mit 92.2 % Homologie auf cDNA-Ebene und 100 % Homologie auf Aminosäure-Ebene. Die isolierte CHO Ub/S27a cDNA ist 508 bp groß und umfaßt die gesamte Kodierregion sowie ein Polyadenylierungssignal im 3' untranslatierten Bereich und zwei einander überlappende Translationsinitiationselemente in der 5' untranslatierten Region (Abb.1). Anhand der cDNA-Sequenz wird auch ersichtlich, daß es sich um ein echtes Fusionsgen handelt, d.h. beide Proteinanteile werden von einem einzigen Gen kodiert. Die Proteinsequenz des resultierenden Fusionsproteins ist hoch konserviert, wobei die ersten 76 Aminosäuren des 156 Aminosäure großen Proteins den Ubiquitinanteil umfassen (Abb.2).

### Beispiel 2: Analyse der Ub/S27a-Genexpression

Das Ausmaß der Ub/S27a-Genexpression wurde in Northern Blot Experimenten, in denen zytoplasmatische Gesamt-RNA von serumkultivierten und serumfrei kultivierten CHO-Zellen verwendet wurde, untersucht.

Die zytoplasmatische RNA wurde von NP40-lysierten Zellen präpariert und durch Phenol/Chloroform-Extraktionen gereinigt (Nicolaides und Stoeckert, 1990). Gesamt-RNA (10 µg) wurde elektrophoretisch in einem Formaldehyd-Agarosegel aufgetrennt, auf eine Nylonmembran (Hybond N, Amersham International plc) transferiert (Sambrook et al., 1989) und durch eine 5minütige UV-Bestrahlung (254 nm) kovalent mit der Nylonmembran vernetzt. Die RNA-Filter wurden über Nacht bei 65°C in einer Lösung bestehend aus 4x SSC, 10x Denhardt, 0.1% SDS und 1 x 10⁶ cpm/ml ³²P-markierter cDNA-Probe hybridisiert. Die cDNA-Fragmente wurden unter Verwendung von "random primer" markiert (Prime a Gene labeling kit; Promega Corporation), wobei die spezifische Aktivität der DNA-Proben 4 -.8 x 10⁸ cpm/µg DNA betrug. Nach der Hybridisierung wurden die Filter zweimal in 0.2x SSC/0.1 % SDS bei 65°C für 20 Minuten gewaschen. Auf die in Haushaltsfolie eingewickelten Filter wurde ein Röntgenfilm (Curix; Agfa-Gevaert N.V.) gelegt und die Autoradiographie fur 3 Stunden bei -70°C durchgeführt.

Sowohl in den unter Standardbedingungen mit Serum kultivierten CHO-Zellen als auch in den an das Wachstum in serumfreiem Medium adaptierten CHO-Zellen ließen sich Ub/S27a-Transkripte mit einer Lange von ungefähr 600 Nukleotiden in großer Zahl und gleichen Mengen nachweisen (Abb.3). Bei den beiden zusätzlichen Transkripten von ungefähr 1500 und 2800 Nukleotiden handelt es sich um Polyubiquitintranskripte, in denen mehrere Ubiquitin-Monomereinheiten miteinander fusioniert sind. Die Polyubiquitintranskripte, die unter diesen Kulturbedingungen zu einem erheblich geringeren Level exprimiert werden, hybridisieren mit dem Ubiquitinanteil der in den Northern Blot Analysen als Probe eingesetzten Ub/S27a cDNA. Der Ub/S27a Transkriptlevel blieb unverändert hoch, wenn die permanent in serumfreiem Medium wachsenden CHO-Zellen für 24 h in serumhaltigem Medium kultiviert wurden (Abb.3). Ein anderes Bild bot sich, wenn die unter Standardbedingungen serumkultivierten CHO-Zellen fur 24 h in serumfreies Medium überführt wurden. Die Zahl der Ub/S27a-Transkripte nahm erheblich ab (Abb.3) Dieses ist darauf zurückzuführen, daß sich der überwiegende Teil der CHO-Zellen während dieser 24stündigen Kultivierungsphase in serumfreiem Medium nicht mehr teilten und zudem eine Reduktion im Zellvolumen aufwiesen. In Zellen, die ein solches Stadium erreicht haben, findet, wenn überhaupt, nur noch eine geringe Transkription statt.

### Beispiel 3: Isolierung und Analyse der Ub/S27a-Promotorregion

Zur Isolierung der Promotorregion des Ub/S27a-Gens wurde zunächst eine genomische CHO-Genbank mit über eine Million rekombinanter Phagenklone hergestellt, wobei genomische DNA von serumkultivierten CHO-Zellen verwendet wurde.

Die genomische DNA wurde von NP40-lysierten Zellen nach einem Protokoll von Nicolaides und Stoeckert gewonnen (Nicolaides & Stoeckert, 1990). Im Gegensatz zu der beschriebenen Ausalzungsmethode wurde die DNA jedoch nach dem Proteinase K1 Verdau dreimal mit Phenol Chloroform extrahiert.

Die DNA-Enden von partiell mit Sau3AI verdauter genomischer DNA mit einer durchschnittlichen Fragmentgröße von 20 kb wurden halbseitig unter Verwendung von dGTP und dATP aufgefüllt und mit Xhol-verdautem Vektor λGEM-12 (halbseitig mit dTTP und dCTP aufgefüllte DNA-Enden; Promega Corporation) ligiert. Zur Verpackung wurden kommerziell erhältliche Extrakte eingesetzt (Gigapack II Plus packaging extracts; Stratagene Cloning Systems).

Diese genomische Genbank wurde lediglich mit dem S27a-Anteil der Ub/S27a-cDNA hybridisiert, um eine Kreuzhybridisierung mit den Polyubiquitingenen zu vermeiden. Einer der isolierten Genomklone mit einer Gesamtlänge von 14 kb enthielt unter anderem die komplette Kodierregion und 2.5 kb der 5' untranslatierten Region. Die Kodierregion wird durch drei Introns mit korrekten Konsensussequenzen an den Exon/Intron- und Intron/Exon-Übergangen unterbrochen (Breathnach & Chambon, 1981), wobei zwei dieser Introns im Ubiquitinanteil und das dritte Intron im S27a-Anteil lokalisiert sind (Abb. 1).

Beide DNA-Stränge der 5' untranslatierten Region wurden komplett sequenziert (Methode s. Beispiel 1). Das geschah durch Sequenzierung von subklonierten Restriktionsfragmenten sowie durch Sequenzierung von überlappenden Deletionsklonen, die durch Exonuklease III Verdau erzeugt worden waren (Abb.4). Die potentielle Promotorregion enthält keine TATA-Box, dafür aber einen CpG-reichen Sequenzbereich (67% GC von - 144 bis + 129), in dem auch die singuläre Bindungsstelle für den Transkriptionsfaktor Sp1 (Dynan & Tjian, 1983) und je eine Restriktionsschnittstelle für EagI und SacII, die für solche GC-reiche Regionen spezifisch sind, liegen, sowie polypyrimidinreiche Sequenzbereiche (Abb. 5).

Zur Lokalisierung des Transkriptionsstartpunktes wurden sowohl Primer-Extension als auch S1 Nuklease-Kartierung an Gesamt-RNA von serumkultivierten CHO-Zellen vorgenommen. Um eine Extension von Polyubiquitin-Transkripten zu vermeiden, wurde für die Primer-Extension ein Primer verwendet, der mit dem S27a-Anteil der Ub/S27a mRNA hybridisierte (komplementär zu den Nukleotiden + 256 - 276 bp in der cDNA-Sequenz).

Ein Oligonukleotid der Sequenz 5'-GTGGTGTAGGACTTCTTCTTC-3', komplementär zu den Nukleotiden + 256 bis + 276 in der Ub/S27a cDNA-Sequenz, wurde mit 5 µg zytoplasmatischer Gesamt-RNA aus serumkultivierten CHO-Zellen hybridisiert und verlängert (Ausubel et al., 1987).

Die zur S 1 Nuklease-Kartierung eingesetzte Einzelstrangprobe, die den Bereich von - 2289 bis + 176 der genomischen Ub/S27a-Sequenz umfaßte, wurde über PCR wie folgt gewonnen. Die 5' untranslatierte Region der Ub/S27a-Genomsequenz (-2289 bis + 240) wurde in 5' - 3' Orientierung in den Vektor pBluescript SK- (Stratagene Cloning Systems) kloniert. Dieses Hybridplasmid wurde in der PCR als Matrize eingesetzt. Zur Amplifizierung wurden ein biotinylierter T3-Promotorprimer und ein mit [γ-³²P] ATP-markierter Ub/S27a-spezifischer Primer (5'-CTCGAGCGTGATCGTTTTCC-3', komplementär zu den Nukleotiden + 157 bis + 176 der Ub/S27a-Genomsequenz) verwendet. Die Gewinnung des zur RNA-Sequenz komplementären Einzelstranges erfolgte durch alkalische Denaturierung des an magnetische Streptavidinbeads gebundenen PCR-Produktes (Dynabeads M-280 Streptavidin Protokoll; Dynal A.S., Norway). 2 x 10⁵ cpm der Einzelstrangprobe wurden mit 5 µg Gesamt-RNA aus serumkultivierten CHO-Zellen über Nacht bei 55°C hybridisiert und die Hybridisierungsprodukte wurden nachfolgend mit S1 Nuklease behandelt (Ausubel et al., 1987).

Das über Primer-Extension erhaltene Produkt wies eine Länge von 304 Nukleotiden auf (Abb.6), womit der Transkriptionsstartpunkt 44 bp stromaufwärts vom Startkodon innerhalb eines Polypyrimidinelements liegen würde (Abb. 5). Dieser Startpunkt konnte durch die S1 Nuklease-Kartierung allerdings nicht verifiziert werden. Über die S1 Nuklease-Kartierung wurden zwei Transkriptionsstartpunkte ermittelt (Abb.7), die 128 bp bzw. 116 bp stromaufwarts vom Startkodon innerhalb einer Polypyrimidinsequenz liegen und deren Positionen im nachfolgenden als Position +1 und +13 angegeben werden (Abb.5, Abb.7). Die wahrscheinlichste Erklärung für die zu beobachtende Diskrepanz zwischen den beiden Kartierungsmethoden ist eine vorzeitige Terminierung der Primer-Extensionsreaktion. Bedingt durch die Positionierung des Primers in der S27a-Sequenz liegt die Länge des zu erwartenden Extensionsproduktes mit über 300 Basen über der optimalen Länge von ungefähr 100 Basen. Je größer die Entfernung zwischen der Primerposition und der gesuchten Transkriptionsstartstelle ist, desto größer ist die Wahrscheinlichkeit eines vorzeitigen Abbruchs der reversen Transkription durch GC-reiche Sequenzen und Sekundärstrukturausbildungen innerhalb der mRNA. Für die S1 Nuklease-Kartierung wurde hingegen eine den gesamten 5' untranslatierten Bereich umfassende Einzelstrangprobe eingesetzt (Sequenzbereich - 2289 bis + 176; Abb.5), die aus einem PCR-Produkt gewonnen wurde, wodurch die bei der Primer-Extension auftretenden Probleme der reversen Transkription von GC-reichen Sequenzen umgangen wurden.

Unsere Untersuchungen der 2.5 kb großen 5' untranslatierten Region zeigten, daß der mögliche Promotor weder eine TATA-Box noch eine CAAT-Box besaß. Dafür aber zeichnete sich die stromaufwärts vom Startkodon gelegene Sequenz durch einen hohen GC-Gehalt aus. Innerhalb dieser Sequenz wurde eine singuläre Bindungsstelle für den Transkriptionsfaktor Sp1 vorgefunden. Über S1 Nuklease-Kartierung wurden zwei prominente Transkriptionsstartpunkte, die innerhalb einer Polypyrimidinsequenz 128 bzw. 116 bp stromaufwärts vom Startkodon lagen, identifiziert

### Beispiel 4: Identifizierung und Eingrenzung des Sequenzbereiches mit Promotoraktivität

### Herstellung von Deletionsklonen durch Exonuklease III Verdau

Über Exonuklease III-Verdau wurde eine Serie von 5' Deletionsklonen hergestellt (Tab.1). Die 5' untranslatierte Region des Ub/S27a-Gens (-2289 bis + 240) wurde dazu in beiden Orientierungen in die HincII-Schnittstelle des Vektors pBluescript SK- (Stratagene Cloning Systems) kloniert. Zur Einführung von unidirektionalen Deletionen wurden diese Hybridplasmide mit KpnI und XhoI verdaut und mit Exonuklease III, wie in dem Protokoll des eingesetzten "Erase-a-base"-Kits beschrieben (Promega Corporation), behandelt. Die erhaltenen DNA-Fragmente wurden als BamHI-Fragmente in die singuläre BamHI-Schnittstelle des Vektors pBL-CAT6 integriert.

Vor Beginn des Exonuklease III-Verdaus kann der Vektor pBluescript Sk- auch durch Einführung von Adaptoren, die geeignete Restriktionsschnittstellen enthalten, modifiziert werden, um nachfolgende Genklonierungsexperimente zu erleichtern. So können neben BamHI auch noch andere Restriktionsenzymschnittstellen zu Klonierungszwecken herangezogen werden, z.B. nach dem Schema

**Tab. 1: Zusammenstellung der durch Exonuklease III- Verdau hergestellten 5' Deletionsklone der Ub/S27a 5' untranslatierten Region**

| 5' Deletionsfragment | 5' Ende des Fragmentes | 3' Ende des Fragmentes |
|---|---|---|
| 1612 bp | Position - 1501 | Position + 111 |
| 806 bp | Position - 695 | Position + 111 |
| 483 bp | Position - 372 | Position + 111 |
| 272 bp | Position - 161 | Position + 111 |
| 156 bp | Position - 45 | Position + 111 |

Die Positionsangaben beziehen sich auf die Numerierung der in Abbildung 5 dargestellten genomischen Ub/S27a-Sequenz.

Alle Deletionsklone haben ein gemeinsames 3' Ende (Position + 111), das zwischen den Transkriptionsstartstellen und dem Startkodon liegt (Abb. 5). Das größte Fragment umfaßt 1.7 kb und das kleinste Fragment 150 bp (Tab.1). Letzteres ist das einzige Fragment, das nicht mehr die singuläre Spl-Bindungsstelle enthält. Diese potentiellen Promotorregionen wurden in den eukaryontischen Expressionsvektor pBL-CAT6 vor das promotorloses CAT-Gen, das als Reportergen diente, kloniert und zur transienten Transfektion von serumkultivierten CHO-Zellen eingesetzt.

### DNA-vermittelte Zelltransfektion und CAT-Assay

Am Vortag der Transfektion wurden 2x10⁵ Zellen pro 20 cm² Schale ausgesät. Die Zellen wurden mit 10 µg Plasmid-DNA (CAT-Reporterkonstrukte) und 500 ng des Plasmides pCMVtklacZ unter Verwendung einer modifizierten Calciumphosphat-Prazipitationsmethode transfiziert (Chen & Okayama, 1987). Die β-Galactosidase-Aktivität des Kontrollvektors pCMVtklacZ wurde dabei zur Bestimmung der Transfektionseffizienz herangezogen. Überschüssiges DNA-Präzipitat wurde nach 4stündiger Inkubation bei 37°C und 5% CO₂ durch Waschen mit PBS entfernt

Nach einer Inkubationsperiode von 48 Stunden wurden die Zellen zunächst mit PBS gewaschen. Danach wurde je 1 ml eiskalter NTE-Puffer (0.1 M NaCl, 10 mM Tris-HCl pH 7.8, 1 mM EDTA) in die Schalen gegeben und die Zellen wurden mit Hilfe eines Schabers von den Schalen abgelöst und in Eppendorfgefäße überführt. Die Zellen wurden durch 3minütige Zentrifugation bei 9000 rpm pelletiert, in 70 µl 0.25 M Tris-HCI pH 7.8 resuspendiert und bei - 70°C gelagert. Fur die Bestimmung der Chloramphenicol-Acetyltransferase-Aktivität nach der Methode von Sleigh wurden jeweils 30 µl der Zellsuspension eingesetzt (Sleigh, 1986). Eine Normalisierung der relativen CAT-Aktivität jeder Transfektion wurde auf der Basis der nach der Methode von Norton und Coffin bestimmten β-Galactosidase-Aktivität vorgenommen (Norton & Coffin, 1985). Für diesen Test wurden jeweils 10 µl der Zellsuspension eingesetzt. In allen Fällen wurde außerdem eine Korrektur bezüglich der eingesetzten Proteinmenge vorgenommen. Die Proteinkonzentration wurde dabei nach der Bradford-Methode bestimmt (Ausubel et al., 1987).

In dem Histogramm in Abbildung 8 sind die Ergebnisse aus vier unabhängig voneinander durchgeführten transienten Expressionsexperimenten dargestellt Zur Kontrolle wurden Plasmide eingesetzt, in denen die CAT-Genexpression durch einen konstitutiven viralen Promotor gesteuert wird. In pBL-CAT5 ist es der Thymidinkinasepromotor des Herpes simplex Virus. In pCMVtkCAT liegt dieser Promotor in Kombination mit einem Enhancer des humanen Cytomegalievirus vor und in pKSSV2CAT handelt es sich um den SV40-Promotor pBL-CAT6 diente als Negativkontrolle und enthält ein promotorloses CAT-Gen.

Mit Ausnahme des 156 bp Fragmentes zeigten alle Ub/S27a-Fragmente, die in 5'-3' Orientierung vor das promotorlose CAT-Reportergen kloniert worden waren, eine starke Promotoraktivität, die 2.5 bis 3mal höher war als die des tk-Promotors des Herpes simplex Virus (Abb.8). Die stärkste Promotoraktivität, die mit der des SV40- Promotors in pKSSV2CAT vergleichbar war, wiesen dabei die Fragmente von 483 bp und 272 bp auf. Lediglich die virale CMV-Enhancer/tk-Promotor-Kombination in pCMVtkCAT resultierte in einer noch um ungefähr 10% höheren CAT-Aktivität. Bei einer bis zur Position - 45 reichenden Deletion (156 bp Fragment), die auch die singulare Sp1-Bindungsstelle umfaßte, war keine CAT-Aktivität mehr feststellbar (Abb.8). Die Ub/S27a 5' untranslatierte Region, die für die Vermittlung einer starken Promotoraktivitat ausreichend ist, läßt sich somit auf den Bereich von - 161, dem 5' Ende des 272 bp Fragmentes, bis - 45, dem 5' Ende des 156 bp Fragmentes, eingrenzen. Innerhalb dieses 117 bp umfassenden Bereiches liegt auch die singuläre Sp1-Bindungsstelle.

Ein unerwartetes Resultat war die Beobachtung, daß die kurzeren Fragmente auch in der 3'-5' Orientierung eine Promotoraktivität aufwiesen (Abb.8) Allerdings ist dieser auf dem Gegenstrang lokalisierte Promotor nicht so stark wie der Ub/S27a-Promotor, sondern um 42% reduziert. Auch hier zeigten die Fragmente von 483 bp und 272 bp die größte Aktivität. Diese Aktivität war mit der des tk-Promotors in pBL-CAT5 vergleichbar. Das zugehörige Gen, dessen Expression von diesem Gegenstrangpromotor aus gesteuert wird, konnte bisher noch nicht identifiziert werden. Es könnte sich also bei dem oben erwähnten 117 bp Promotorbereich sogar um eine bidirektionale Promotorregion handeln.

Zusammenfassend kann gesagt werden, daß mit dem von den Erfindern der vorliegenden Erfindung isolierten Ub/S27a-Promotor aus CHO-Zellen erstmals ein sehr starker konstitutiver homologer Promotor des Hamsters bereitgestellt wird. Untersuchungen an stabil transfizierten serumkultivierten CHO-Zellen belegen, daß der Ub/S27a-Promotor auch nach der Integration in das Zellgenom äußerst aktiv ist und für eine sehr starke Expression des CAT-Reportergenes sorgt.

### Beispiel 5: Transiente Expression eines Reportergens unter der Kontrolle des Ub/S27a-Promotors in BHK-Zellen

CAT-Reporterkonstrukte, die verschiedene Fragmente der Ub/S27a-Promotorregion enthielten, wurden analog in BHK-21-Zellen (ATCC CCL 10) eingebracht und die CAT-Aktivität der Transfektanten gemessen. Abb. 9 zeigt, daß mit den erfindungsgemäßen Promotorsequenzen auch in BHK-Zellen eine außerordentlich hohe Expressionsrate erzielt werden kann.

### Beispiel 6: Stabile Expression eines Reportergens unter der Kontrolle des Ub/S27a-Promotors in CHO-Zellen

Stabile Transfektanten wurden wie folgt hergestellt. Am Vortag der Transfektion wurden 200.000 Zellen pro 20-cm²-Schale ausgesät. Die Zellen wurden mit 10 µg Plasmid-DNA (CAT-Reporterkonstrukte) und 500 ng des Plasmides pSV2pac (Vara *et al.,* 1986) unter Verwendung einer modifizierten Calciumphosphat-Präzipitationsmethode transfiziert (Chen & Okayama, 1987). Das Plasmid pSV2pac trägt das für die Puromycin-N-Acetyltransferase (pac) kodierende Gen und vermittelt somit Resistenz gegen das die Proteinbiosynthese hemmende Antibiotikum. Überschüssiges DNA-Präzipitat wurde nach 4stündiger Inkubation bei 37°C und 5% CO₂ durch Waschen mit PBS entfernt. Nach 24 weiteren Stunden wurde mit der Selektion der Transfektanten durch Zugabe von 10 µg/ml Puromycin gestartet. Alle zwei bis drei Tage erfolgte ein Mediumwechsel mit Puromycin-haltigem Medium. Über Verdünnungsklonierung der selektionierten Transfektanten wurden Einzelzellklone gewonnen.

Abb. 10-12 zeigen CAT-Expressionsraten verschiedener Klone stabiler Transfektanten relativ zu pCMVtkCAT cl.6 (Klon 6, der pCMVtkCAT-transfizierte Zellklon mit der höchsten CAT-Aktivität).

### Literatur

Adams, S.M., Sharp, M.G., Walker, R.A., Brammar, W.J. & Varley, J.M. (1992) Differential expression of translation-associated genes in benign and malignant human breast tumours
   Br.J.Cancer 65, 65 - 71 1
Ausubel, F.M., Brent, R., Kingston, R.E., Moore, D.D., Smith, J.A., Seidman, J.G. & Struhl, K (1987)
   Current protocols in molecular biology
   Greene Publishing Associates and Wiley-Interscience
Baker, R.T. & Board, P.G. (1991)
   The human ubiquitin-52 amino acid fusion protein gene shares several structural features with mammalian ribosomal protein genes
   Nucleic Acids Research 19, 1035 - 1040
Boshart, M., Klüppel, M., Schmidt, A. , Schütz, G. & Luckow, B. (1992)
   Reporter constructs with low background activity utilizing the cat gene
   Gene 110, 129 - 130
Breathnach, R & Chambon, P. (1981)
   Organization and expression of eukaryotic split genes coding for proteins
   Ann.Rev.Biochem. 50, 349 - 383
Chen, C. & Okayama, H. (1987)
   High-efficiency transformation of mammalian cells by plasmid DNA
   J. Mol. Cell. Biol. 7, 2745 - 2752
Ciechanover, A. (1994)
   The ubiquitin-proteasome proteolytic pathway
   Cell 79, 13 - 21
Dynan, W.S. & Tjian, R. (1983)
   The promoter-specific transcription factor Sp1 binds to upstream sequences in the SV40 early promoter
   Cell 35, 79 - 87
Faisst, S. & Meyer, S. (1992)
   Compilation of vertebrate-encoded transcription factors
   Nucleic Acids Research 20, 3 - 26
Finley, D., Bartel, B. & Varshavsky, A. (1989)
   The tails of ubiquitin precursors are ribosomal proteins whose fusion to ubiquitin facilitates ribosome biogenesis
   Nature 333, 394 - 401
Fornace, A.J., Alama, I., Hollander, M.C. & Lamoreaux, E. (1989)
   Ubiquitin mRNA is a major stress-induced transcript in mammalian cells
   Nucleic Acids Research 17, 1215 - 1230
Huxley, C. & Fried, M. (1990)
   The mouse rpL7a gene is typical of other ribosomal protein genes in it's 5' region but differs in being located in a tight cluster of CpG-rich islands
   Nucleic Acids Research 18, 5353 - 5357
Jentsch, S., Seufert, W. & Hauser, H.-P. (1991)
   Genetic analysis of the ubiquitin system
   Biochimica et Biophysica Acta 1089, 127 - 139
Kaufman R. J. (1987)
   High level production of proteins in mammalian cells
   In: Genetic Engineering: Principles and methods (Hrsg. J K Setlow), Bd. 9, S. 155
   Plenum Publishing, New York
Luckow, B., Schütz, G. (1987)
   CAT constructions with multiple unique restriction sites for the functional analysis of eucaryotic promoters and regulatory elements
   Nucleic Acids Res. 15, 5490
Luo, X. & Kim, K.-H. (1990)
   An enhancer element in the house-keeping promoter for acetyl-CoA carboxylase gene
   Nucleic Acids Research 18, 3249 - 3254
Meyer, J., Nick, S., Stamminger, T., Grummt, F., Jahn, G. & Lipps, H.J. (1993)
   Inhibition of HIV-1 replication by a high-copy-number vector expressing antisense RNA for reverse transcriptase
   Gene 129, 263-268
Nicolaides, N.C. & Stoeckert, C.J. (1990)
   A simple, efficient method for the separate isolation ofRNA and DNA from the same cells
   BioTechniques 8, 154 - 155
Norton, P.A. & Coffin, J.M. (1985)
   Bacterial β-galactosidase as a marker of Rous Sarcoma virus gene expression and replication
   Mol. Cell. Biol. 5, 281 - 290
Redman, K.L. & Rechsteiner, M. (1989)
   Identification ofthe long ubiquitin extension as ribosomal protein S27a
   Nature 338,438 - 440
Sambrook, J., Fritsch, E.F. & Maniatis, T. (1989)
   Molecular cloning A Laboratory Manual
   Cold Spring Harbor Laboratory, Cold Spring Harbor, NY
Schlesinger, M.J. & Bond, U. (1987)
   Ubiquitin genes
   Oxf. Survey Euk. Genes 4, 77 - 91
Shimbara, N., Sato, C., Takashina, M., Tanaka, T., Tanaka, K. & Ichihara, A. (1993)
   Down-regulation of ubiquitin gene expression during differentiation of human leukemia cells FEBS Letters 322, 235 - 239
Sleigh, M.J. (1986)
   A nonchromatographic assay for expression of the chloramphenicol acetyl transferase gene in eukaryotic cells
   Anal. Biochem. 156, 252 - 256
Urlaub, G. & Chasin, L.A. (1980)
   Isolation of chinese hamster cell mutants deficient in dihydrofolate reductase activity
   Proc. Natl. Acad. Sci. USA 77, 4216 - 4220
Tsonis, P.A., Manes, T., Millan, J.L. & Goetinck, P.F. (1988)
   CAT constructs with convenient sites for cloning and generating deletions
   Nucleic Acids Research 16, 7745
Vara, J.A., Portela, A., Ortin, J. & Jiménez, A. (1986)
   Expression in mammalian cells of a gene from Streptomyces alboniger conferring puromycin resistance
   Nucleic Acids Research 14, 4617 - 4624
Wegner, M., Zastrow, G., Klavinius, A., Schwender, S., Müller, F., Luksza, H., Hoppe, J., Wienberg, J. & Grummt, F. (1989)
   Cis-acting sequences from mouse rDNA promote plasmid DNA amplification and persistence in mouse cells: implication of HMG-I in their function
   Nucleic Acids Research 17, 9909 - 9932
Wiborg, O., Pedersen, M.S., Wind, A., Berglund, L.E., Marcker, K.A. & Vuust, J. (1985)
   The human ubiquitin multigene family: some genes contain multiple directly repeated ubiqutin coding sequences
   EMBO J. 4, 755 - 759
Wong, J.M., Mafune, K., Yow, H., Rivers, E.N., Ravikumar, T.S., Steele, G.D. & Chen, L.B. (1993)
   Ubiquitin-ribosomal protein S27a gene overexpressed in human colorectal carcinoma is an early growth response gene
   Cancer Research 53, 1916 - 1920

## Patentansprüche

1. Nukleinsäuremolekül enthaltend die Promotorsequenz aus dem Ubiquitin-S27a-Gen des Hamsters von Position 2129 bis 2400 gemäß SEQ ID NO:5 oder eine Sequenz, die durch Substitution, Insertion oder Deletion von einer, zwei, oder drei Basen von dieser ableitbar ist, ohne dass durch diese Substitution, Insertion oder Deletion die Promotoraktivität um mehr als 50% des Wertes erniedrigt wird, der für einen Promotor mit der Sequenz von Position 2129 bis 2400 gemäß SEQ ID NO:5 erhalten wird.

2. Nukleinsäuremolekül nach Anspruch 1 **dadurch gekennzeichnet, dass** es die Promotorsequenz aus dem Ubiquitin-S27a-Gen des Hamsters von Position 1918 bis 2400 gemäß SEQ ID NO:5 oder eine Sequenz, die durch Substitution, Insertion oder Deletion von einer, zwei, oder drei Basen von dieser ableitbar ist, enthält, ohne dass durch diese Substitution, Insertion oder Deletion die Promotoraktivität um mehr als 50% des Wertes erniedrigt wird, der für einen Promotor mit der Sequenz von Position 2129 bis 2400 gemäß SEQ ID NO:5 erhalten wird.

3. Nukleinsäuremolekül nach Anspruch 1 oder 2, **dadurch gekennzeichnet, dass** es die Promotorsequenz aus dem Ubiquitin-S27a-Gen des Hamsters gemäß SEQ ID NO:5 oder eine Sequenz, die durch Substitution, Insertion oder Deletion von einer, zwei, oder drei Basen von dieser ableitbar ist, enthält, ohne dass durch diese Substitution, Insertion oder Deletion die Promotoraktivität um mehr als 50% des Wertes erniedrigt wird, der für einen Promotor mit der Sequenz von Position 2129 bis 2400 gemäß SEQ ID NO:5 erhalten wird.

4. Nukleinsäuremolekül nach einem der Ansprüche 1 bis 3, **dadurch gekennzeichnet, dass** es einen oder mehrere Enhancer enthält, der/die mit der Promotorsequenz aus dem Ubiquitin-S27a-Gen des Hamsters funktionell verknüpft ist/sind.

5. Nukleinsäuremolekül nach einem der Ansprüche 1 bis 4, **dadurch gekennzeichnet, dass** die Promotorsequenzen mit einem Gen funktionell verknüpft sind.

6. Nukleinsäuremolekül nach Anspruch 5, **dadurch gekennzeichnet, dass** das besagte Gen unter der Kontrolle der Promotorsequenzen exprimiert wird.

7. Nukleinsäuremolekül nach einem der Ansprüche 5 oder 6, **dadurch gekennzeichnet, dass** das besagte Gen für Gewebeplasminogenaktivator, *second-*generation-Gewebeplasminogenaktivator, Interferon, Tumornekrosefaktor, Erythropoietin, Granulozyten-Kolonie-stimulierenden Faktor, Mangan-Superoxiddismutase, eine Immunglobulinkette, die variable Region einer Immunglobulinkette, eine humaniserte Immunglobulinkette, die variable Region einer humanisierten Immunglobulinkette, einen single-chain-Antikörper und/oder einen Antikörper, der für variantes CD44 spezifisch ist, kodiert.

8. Nukleinsäuremolekül nach einem der Ansprüche 1 bis 7, **dadurch gekennzeichnet, dass** es ein Expressionsvektor ist.

9. Wirtszelle, in die ein Nukleinsäuremolekül nach einem der Ansprüche 1 bis 8 eingeführt wurde.

10. Verfahren zur Herstellung eines heterologen Genprodukts in einer eukaryontischen Wirtszelle, **dadurch gekennzeichnet, dass** ein Nukleinsäuremolekül gemäß einem der Ansprüche 1 bis 8 in die eukaryontische Wirtszelle eingeführt wird, die Wirtszelle kultiviert und das synthetisierte Genprodukt isoliert wird.

11. Verfahren nach Anspruch 10, **dadurch gekennzeichnet, dass** die Wirtszelle eine Hamsterzelle ist.

12. Verfahren nach Anspruch 11, **dadurch gekennzeichnet, dass** die Hamsterzelle eine CHO-Zelle ist.

13. Verfahren nach einem der Ansprüche 10 bis 12, **dadurch gekennzeichnet, dass** das heterologe Gen für Gewebeplasminogenaktivator, *second-generation-*Gewebeplasminogenaktivator, Interferon, Tumornekrosefaktor, Erythropoietin, Granulozyten-Kolonie-stimulierenden Faktor, Mangan-Superoxiddismutase, eine Immunglobulinkette, die variable Region einer Immunglobulinkette, eine humaniserte Immunglobulinkette, die variable Region einer humanisierten Immunglobulinkette, einen single-chain-Antikörper und/oder einen Antikörper, der für variantes CD44 spezifisch ist, kodiert.

14. Verwendung eines Nukleinsäuremoleküls enthaltend eine Promotersequenz nach einem der Ansprüche 1 bis 8 zur Herstellung eines heterologen Genprodukts in kultivierten Hamsterzellen, vorzugsweise CHO-Zellen.

## Claims

1. Nucleic acid molecule containing the promoter sequence from the ubiquitin-S27a gene of the hamster from position 2129 to 2400 according to SEQ ID NO:5 or a sequence which can be derived therefrom by substitution, insertion or deletion of one, two or three bases without the promoter activity being reduced by this substitution, insertion or deletion by more than 50% of the value which is obtained for a promoter with the sequence from position 2129 to 2400 according to SEQ ID NO:5.

2. Nucleic acid molecule according to claim 1, **characterised in that** it contains the promoter sequence from the ubiquitin-S27a gene of the hamster from position 1918 to 2400 according to SEQ ID NO:5 or a sequence which can be derived therefrom by substitution, insertion or deletion of one, two or three bases without the promoter activity being reduced by this substitution, insertion or deletion by more than 50% of the value which is obtained for a promoter with the sequence from position 2129 to 2400 according to SEQ ID NO:5.

3. Nucleic acid molecule according to claim 1 or 2, **characterised in that** it contains the promoter sequence from the ubiquitin-S27a gene of the hamster according to SEQ ID NO:5 or a sequence which can be derived therefrom by substitution, insertion or deletion of one, two or three bases without the promoter activity being reduced by this substitution, insertion or deletion by more than 50% of the value which is obtained for a promoter with the sequence from position 2129 to 2400 according to SEQ ID NO:5.

4. Nucleic acid molecule according to one of claims 1 to 3, **characterised in that** it contains one or more enhancers which is/are functionally connected to the promoter sequence from the ubiquitin-S27a gene of the hamster.

5. Nucleic acid molecule according to one of claims 1 to 4, **characterised in that** the promoter sequences are functionally connected to a gene.

6. Nucleic acid molecule according to claim 5, **characterised in that** said gene is expressed under the control of the promoter sequences.

7. Nucleic acid molecule according to one of claims 5 or 6, **characterised in that** said gene codes for tissue plasminogen activator, second-generation tissue plasminogen activator, interferon, tumour necrosis factor, erythropoietin, granulocyte-colony-stimulating factor, manganese-superoxide dismutase, an immunoglobulin chain, the variable region of an immunoglobulin chain, a humanised immunoglobulin chain, the variable region of a humanised immunoglobulin chain, a single-chain antibody and/or an antibody which is specific to variant CD44.

8. Nucleic acid molecule according to one of claims 1 to 7, **characterised in that** it is an expression vector.

9. Host cell into which a nucleic acid molecule according to one of claims 1 to 8 has been introduced.

10. Process for preparing a heterologous gene product in a eukaryotic host cell, **characterised in that** a nucleic acid molecule according to one of claims 1 to 8 is introduced into the eukaryotic host cell, the host cell is cultivated and the synthesised gene product is isolated.

11. Process according to claim 10, **characterised in that** the host cell is a hamster cell.

12. Process according to claim 11, **characterised in that** the hamster cell is a CHO cell.

13. Process according to one of claims 10 to 12, **characterised in that** the heterologous gene codes for tissue plasminogen activator, second-generation tissue plasminogen activator, interferon, tumour necrosis factor, erythropoietin, granulocyte-colony-stimulating factor, manganese-superoxide dismutase, an immunoglobulin chain, the variable region of an immunoglobulin chain, a humanised immunoglobulin chain, the variable region of a humanised immunoglobulin chain, a single-chain antibody and/or an antibody which is specific to variant CD44.

14. Use of a nucleic acid molecule containing a promoter sequence according to one of claims 1 to 8, for preparing a heterologous gene product in cultivated hamster cells, preferably CHO cells.

## Revendications

1. Molécule d'acide nucléique contenant la séquence promotrice du gène d'ubiquitine S27a du hamster des positions 2129 à 2400 selon SEQ ID NO : 5 ou une séquence qui peut être déduite de celle-ci par substitution, insertion ou délétion d'une, deux ou trois bases, sans que l'activité promotrice soit réduite par cette substitution, insertion ou délétion de plus de 50 % de la valeur qui est obtenue pour un promoteur ayant la séquence des positions 2129 à 2400 selon SEQ ID NO : 5.

2. Molécule d'acide nucléique selon la revendication 1 **caractérisée en ce qu'**elle contient la séquence promotrice du gène d'ubiquitine S27a du hamster des positions 1918 à 2400 selon SEQ ID NO : 5 ou une séquence qui peut être déduite de celle-ci par substitution, insertion ou délétion d'une, deux ou trois bases, sans que l'activité promotrice soit réduite par cette substitution, insertion ou délétion de plus de 50 % de la valeur qui est obtenue pour un promoteur ayant la séquence des positions 2129 à 2400 selon SEQ ID NO : 5.

3. Molécule d'acide nucléique selon la revendication 1 ou 2 **caractérisée en ce qu'**elle contient la séquence promotrice du gène d'ubiquitine S27a du hamster selon SEQ ID NO : 5 ou une séquence qui peut être déduite de celle-ci par substitution, insertion ou délétion d'une, deux ou trois bases, sans que l'activité promotrice soit réduite par cette substitution, insertion ou délétion de plus de 50 % de la valeur qui est obtenue pour un promoteur ayant la séquence des positions 2129 à 2400 selon SEQ ID NO : 5.

4. Molécule d'acide nucléique selon l'une des revendications 1 à 3 **caractérisée en ce qu'**elle contient un ou plusieurs enhancers qui est/sont relié(s) fonctionnellement à la séquence promotrice du gène d'ubiquitine S27a du hamster.

5. Molécule d'acide nucléique selon l'une des revendications 1 à 4 **caractérisée en ce que** les séquences promotrices sont reliées fonctionnellement à un gène.

6. Molécule d'acide nucléique selon la revendication 5 **caractérisée en ce que** ledit gène est exprimé sous le contrôle des séquences promotrices.

7. Molécule d'acide nucléique selon l'une des revendications 5 ou 6 **caractérisée en ce que** ledit gène code l'activateur du plasminogène tissulaire, l'activateur du plasminogène tissulaire *de seconde génération,* l'interféron, le facteur de nécrose des tumeurs, l'érythropoïétine, le facteur stimulant les colonies de granulocytes, la manganèse-superoxyde dismutase, une chaîne d'immunoglobuline, la région variable d'une chaîne d'immunoglobuline, une chaîne d'immunoglobuline humanisée, la région variable d'une chaîne d'immunoglobuline humanisée, un anticorps single-chain et/ou un anticorps qui est spécifique de CD44 variant.

8. Molécule d'acide nucléique selon l'une des revendications 1 à 7 **caractérisée en ce que** c'est un vecteur d'expression.

9. Cellule hôte dans laquelle une molécule d'acide nucléique selon l'une des revendications 1 à 8 a été introduite.

10. Procédé de production d'un produit génique hétérologue dans une cellule hôte eucaryote **caractérisé en ce qu'**une molécule d'acide nucléique selon l'une des revendications 1 à 8 est introduite dans la cellule hôte eucaryote, la cellule hôte est cultivée et le produit génique synthétisé est isolé.

11. Procédé selon la revendication 10 **caractérisé en ce que** la cellule hôte est une cellule de hamster.

12. Procédé selon la revendication 11 **caractérisé en ce que** la cellule de hamster est une cellule CHO.

13. Procédé selon l'une des revendications 10 à 12 **caractérisé en ce que** le gène hétérologue code l'activateur du plasminogène tissulaire, l'activateur du plasminogène tissulaire *de seconde génération,* l'interféron, le facteur de nécrose des tumeurs, l'érythropoïétine, le facteur stimulant les colonies de granulocytes, la manganèse-superoxyde dismutase, une chaîne d'immunoglobuline, la région variable d'une chaîne d'immunoglobuline, une chaîne d'immunoglobuline humanisée, la région variable d'une chaîne d'immunoglobuline humanisée, un anticorps single-chain et/ou un anticorps qui est spécifique de CD44 variant.

14. Utilisation d'une molécule d'acide nucléique contenant une séquence promotrice selon l'une des revendications 1 à 8 pour la production d'un produit génique hétérologue dans des cellules de hamster cultivées, de préférence des cellules CHO.


### SEQUENZPROTOKOLL

(1) ALLGEMEINE ANGABEN:
   (i) ANMELDER:
      (A) NAME: Boehringer Ingelheim International GmbH
      (B) STRASSE: Binger Str.
      (C) ORT: Ingelheim
      (E) LAND: Deutschland
      (F) POSTLEITZAHL: 55216
      (G) TELEFON: 06132-772770
      (H) TELEFAX: 06132-774377
   (ii) BEZEICHNUNG DER ERFINDUNG: Starker homologer Promotor aus Hamster
   (iii) ANZAHL DER SEQUENZEN: 8
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 508 Basenpaare
      (B) ART: Nucleotid
      (C) STRANGFORM: beides
      (D) TOPOLOGIE: beides
   (ii) ART DES MOLEKÜLS: cDNA zu mRNA
   (xi) SEQUENZBESCHREIBUNG: SEQ ID NO: 1:
   TGGAACCGCC GCCAAGATGC AGATTTTCGT GAAGACCCTT ACGGGGAAAA CGATCACGCT   60
   CGAGGTTGAA CCCTCGGACA CTATAGAAAA TGTAAAGGCC AAGATCCAGG ATAAGGAAGG  120
   AATTCCTCCT GACCAGCAGA GGCTGATCTT TGCTGGTAAG CAACTGGAAG ATGGCCGTAC  180
   TTTGTCTGAC TACAACATCC AAAAGGAGTC CACCCTTCAT CTTGTGTTGA GACTTCGTGG  240
   TGGTGCTAAG AAGAGGAAGA AGAAGTCCTA CACCACTCCC AAGAAGAATA AGCATAAGAG  300
   AAAGAAGGTT AAGTTGGCTG TGCTGAAGTA CTATAAGGTG GATGAAAATG GCAAAATTAG  360
   TCGCCTTCGT CGAGAGTGTC CATCTGATGA GTGTGGTGCT GGAGTTTTCA TGGCTAGCCA  420
   TTTTGACAGA CATTACTGTG GCAAGTGTTG TCTGACTTAC TGCTTCAACA AACCAGAAGA  480
   CAAGTAGTTG TGTATGAATA AATAAAAA                                      508
(2) ANGABEN ZU SEQ ID NO: 2:
   (i) SEQUENZKENNZEICHEN:
      (A) LÄNGE : 487 Basenpaare
      (B) ART: Nucleotid
      (C) STRANGFORM: beides
      (D) TOPOLOGIE: beides
   (ii) ART DES MOLEKÜLS: cDNA
   (xi) SEQUENZBESCHREIBUNG: SEQ ID NO: 2:
   TGGAGCCGCA ACCAAAATGC AGATTTTCGT GAAAACCCTT ACGGGGAAGA CCATCACCCT  60
   CGAGGTTGAA CCCTCGGATA CGATAGAAAA TGTAAAGGCC AAGATCCAGG ATAAGGAAGG 120
   AATTCCTCCT GATCAGCAGA GACTGATCTT TGCTGGCAAG CAGCTAGAAG ATGGACGTAC 180
   TTTGTCTGAC TACAATATTC AAAAGGAGTC TACTCTTCAT CTTGTGTTGA GACTTCGTGG 240
   TGGTGCTAAG AAAAGGAAGA AGAAGTCTTA CACCACTCCC AAGAAGAATA AGCACAAGAG 300
   AAAGAAGGTT AAGCTGGCTG TCCTGAAATA TTATAAGGTG GATGAGAATG GCAAAATTAG 360
   TCGCCTTCGT CGAGAGTGCC CTTCTGATGA ATGTGGTGCT GGGGTGTTTA TGGCAAGTCA 420
   CTTTGACAGA CATTATTGTG GCAAATGTTG TCTGACTTAC TGTTTCAACA AACCAGAAGA 480
   CAAGTAA                                                           487
(2) ANGABEN ZU SEQ ID NO: 3:
   (i) SEQUENZKENNZEICHEN:
      (A) LÄNGE: 156 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 3:
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu His Leu Val Leu Arg Leu Arg Gly Gly Ala Lys Lys Arg
   65                  70                  75                  80
   Lys Lys Lys Ser Tyr Thr Thr Pro Lys Lys Asn Lys His Lys Arg Lys
                   85                  90                  95
   Lys Val Lys Leu Ala Val Leu Lys Tyr Tyr Lys Val Asp Glu Asn Gly
               100                 105                 110
   Lys Ile Ser Arg Leu Arg Arg Glu Cys Pro Ser Asp Glu Cys Gly Ala
           115                 120                 125
   Gly Val Phe Met Ala Ser His Phe Asp Arg His Tyr Cys Gly Lys Cys
       130                 135                 140
   Cys Leu Thr Tyr Cys Phe Asn Lys Pro Glu Asp Lys
   145                 150                 155
(2) ANGABEN ZU SEQ ID NO: 4:
   (i) SEQUENZKENNZEICHEN:
      (A) LÄNGE: 156 Aminos"uren
      (B) ART: Aminos"ure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 4:
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu His Leu Val Leu Arg Leu Arg Gly Gly Ala Lys Lys Arg
   65                  70                  75                  80
   Lys Lys Lys Ser Tyr Thr Thr Pro Lys Lys Asn Lys His Lys Arg Lys
                   85                  90                  95
   Lys Val Lys Leu Ala Val Leu Lys Tyr Tyr Lys Val Asp Glu Asn Gly
                       100                 105                 110
   Lys Ile Ser Arg Leu Arg Arg Glu Cys Pro Ser Asp Glu Cys Gly Ala
               115                 120                 125
   Gly Val Phe Met Ala Ser His Phe Asp Arg His Tyr Cys Gly Lys Cys
           130                 135                 140
   Cys Leu Thr Tyr Cys Phe Asn Lys Pro Glu Asp Lys
   145                 150                 155
(2) ANGABEN ZU SEQ ID NO: 5:
   (i) SEQUENZKENNZEICHEN:
      (A) LANGE: 2529 Basenpaare
      (B) ART: Nucleotid
      (C) STRANGFORM: beides
      (D) TOPOLOGIE: linear
   (ii) ART DES MOLEKÜLS: Genom-DNA
   (xi) SEQUENZBESCHREIBUNG: SEQ ID NO: 5:
   GATCTCCAGG ACAGCCATGG CTATTACACA GAGAAACCCT GTCTGGAAAA ACAAAAAATT   60
   AGTGTCCATG TGTAAATGTG TGGAGTATGC TTGTCATGCC ACATACAGAG GTAGAGGGCA  120
   GTTTATGGGA GTCAGTTCCT ATTCTTCCTT TATGGGGGAC CTGGGGACTG AACTCAGGTC  180
   ATCAGGCTTG GCAGAAAGTG CATTAGCTCA CGGAGCCTTA TCATTGGCGA AAGCTCTCTC  240
   AAGTAGAAAA TCAATGTGTT TGCTCATAGT GCAATCATTA TGTTTCGAGA GGGGAAGGGT  300
   ACAATCGTTG GGGCATGTGT GGTCACATCT GAATAGCAGT AGCTCCCTAG GAGAATTCCA  360
   AGTTCTTTGG TGGTGTATCA ATGCCCTTAA AGGGGTCAAC AACTTTTTTT CCCTCTGACA  420
   AAACTATCTT CTTATGTCCT TGTCCCTCAT ATTTGAAGTA TTTTATTCTT TGCAGTGTTG  480
   AATATCAATT CTAGCACCTC AGACATGTTA GGTAAGTACC CTACAACTCA GGTTAACTAA  540
   TTTAATTTAA CTAATTTAAC CCCAACACTT TTTCTTTGTT TATCCACATT TGTGGAGTGT  600
   GTGTGTGTGT GTGTGTGTGT GTGTGTGTGT GTGTGTGTGT GTGTGTGTGT GTGTGTGTGC  660
   CGCGCGCGCT CGGATCATTC TACCTTTTGT TTAAAAAATG TTAGTCCAGG GGTGGGGTGC  720
   ACTGTGAAAG TCTGAGGGTA ACTTGCTGGG GTCAGTTCTT TCCACTATAG GACAGAACTC  780
   CAGGTGTCAA CTCTTTACTG ACAGAACCAT CCAAATAGCC CTATCTAATT TTAGTTTTTT  840
   ATTTATTTAT TTTTTGTTTT TCGAGACAGG GTTTCTCTGT GGCTTTGGAG GCTGTCCTGG  900
   AACTAGCTCT TGTAGACCAG GCTGGTCTCG AACTCAGAGA TCCACCTGCC TCTGCCTCCT  960
   GAGTGCTGGG ATTAAAGGCA TGCGCCACCA ACGCTTGGCT CTACCTAATT TTAAAAGAGA 1020
   TTGTGTGTCA CAAGGGTGTC ATGTCGCCCT GCAACCACCC CCCCCCCAAA AAAAAAAAAA 1080
   AAAAACTTCA CTGAAGCTGA AGCACGATGA TTTGGTTACT CTGGCTGGCC AATGAGCTCT 1140
   AGGGAGTCTC CTGTCAAACA GAATCTCAAC AGGCGCAGCA GTCTTTTTTA AAGTGGGGTT 1200
   ACAACACAGG TTTTTGCATA TCAGGCATTT TATCTAAGCT ATTTCCCAGC CAAAAATGTG 1260
   TATTTTGGAG GCAGCAGAGC TAATAGATTA AAATGAGGGA AGAGCCCACA CAGGTTATTA 1320
   GGAAGATAAG CATCTTCTTT ATATAAAACA AAACCAAACC AAACTGGAGG AGGTCTACCT 1380
   TTAGGGATGG AAGAAAAGAC ATTTAGAGGG TGCAATAGAA AGGGCACTGA GTTTGTGAGG 1440
   TGGAGGACTG GGAGAGGGCG CAACCGCTTT AACTGTCCTG TTTTGCCTAT TTTTTGGGGA 1500
   CAGCACATGT TCCTATTTTT CCCAGGATGG GCAATCTCCA CGTCCAAACT TGCGGTCGAG 1560
   GACTACAGTC ATTTTGCAGG TTTCCTTACT GTATGGCTTT TAAAACGTGC AAAGGTGACC 1620
   ATTAACCGTT TCACGCTGGG AGGGCACGTG CGGCTCAGAT GCTTCCTCTG ACTGAGGGCC 1680
   AGGAGGGGGC TACACGGAAG AGGCCACACC CGCACTTGGG AAGACTCGAT TTGGGCTTCA 1740
   GCTGGCTGAG ACGCCCCAGC AGGCTCCTCG GCTACACCTT CAGCCCCGAA TGCCTTCCGG 1800
   CCCATAACCC TTCCCTTCTA GGCATTTCCG GCGAGGACCC ACCCTCGCGC CAAACATTCG 1860
   GCCCCATCCC CCGGTCCTCA CCTGAATCTC TAACTCTGGA CTCCAGAGTT TAGAGACTAT 1920
   AACCAGATAG CCCGGATGTG TGGAACTGCA TCTTGGGACG AGTAGTTTTA GCAAAAAGAA 1980
   AGCGACGAAA AACTACAATT CCCAGACAGA CTTGTGTTAC CTCTCTTCTC ATGCTAAACA 2040
   AGCCCCCTTT AAAGGAAAGC CCCTCTTAGT CGCATCGACT GTGTAAGAAA GGCGTTTGAA 2100
   ACATTTTAAT GTTGGGCACA CCGTTTCGAG GACCGAAATG AGAAAGAGCA TAGGGAAACG 2160
   GAGCGCCCGA GCTAGTCTGG CACTGCGTTA GACAGCCGCG GTCGTTGCAG CGGGCAGGCA 2220
   CTTGCGTGGA CGCCAAGGGG CGGGTCTTTC GGCCGGGAAG CCCCGTTGGT CCGCGCGGCT 2280
   CTTCCTTTCC GATCCGCCAT CCGTGGTGAG TGTGTGCTGC GGGCTGCCGC TCCGGCTTGG 2340
   GGCTTCCCGC GTCGCTCTCA CCCTGGTCGG CGGCTCTAAT CCGTCTCTTT TCGAATGTAG 2400
   GTGGAACCGC CGCCAAGATG CAGATTTTCG TGAAGACCCT TACGGGGAAA ACGATCACGC 2460
   TCGAGGTACG AACCAGGTGG CGTGAGAAGC GAAGGCCTGC CAGAGGCCCT CTATGCTCGC 2520
   TTAAAGCTT 2529
(2) ANGABEN ZU SEQ ID NO: 6:
   (i) SEQUENZKENNZEICHEN:
      (A) LÄNGE: 117 Basenpaare
      (B) ART: Nucleotid
      (C) STRANGFORM: beides
      (D) TOPOLOGIE: linear
   (ii) ART DES MOLEKÜLS: Genom-DNA
   (xi) SEQUENZBESCHREIBUNG: SEQ ID NO: 6:
      AGGACCGAAA TGAGAAAGAG CATAGGGAAA CGGAGCGCCC GAGCTAGTCT GGCACTGCGT 60 60
      TAGACAGCCG CGGTCGTTGC AGCGGGCAGG CACTTGCGTG GACGCCAAGG GGCGGGT 117
(2) ANGABEN ZU SEQ ID NO: 7:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "primer"
   (xi) SEQUENZBESCHREIBUNG: SEQ ID NO: 7:
      GTGGTGTAGG ACTTCTTCTT C 21
(2) ANGABEN ZU SEQ ID NO: 8:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleins,,ure
      (A) BESCHREIBUNG: /desc = "primer"
   (xi) SEQUENZBESCHREIBUNG: SEQ ID NO: 8:
      CTCGAGCGTG ATCGTTTTCC 20